# EUROPÄISCHE PATENTSCHRIFT

(11) **EP 1 337 219 B1**
(45) Veröffentlichungstag und Bekanntmachung des Hinweises auf die Patenterteilung: **02.11.2006**
(21) Anmeldenummer: 01997273.6
(22) Anmeldetag: 23.11.2001
(51) Int. Cl.: A61K 8/04, B01J 13/00

(54) **PHASENTRANSFER VON NANOPARTIKELN**
PHASE TRANSFER OF NANOPARTICLES
TRANSFERT DE PHASE DE NANOPARTICULES

(30) Priorität: 24.11.2000 DE 10058544; 10.07.2001 DE 10132564; 27.07.2001 DE 10136583
(43) Veröffentlichungstag der Anmeldung: 27.08.2003
(73) Patentinhaber: Nanogate AG, 66121 Saarbrücken (DE)
(72) Erfinder: CARUSO, Frank, Victoria 3010 (AU); GITTINS, David, Moreton CH46 9QS (GB)
(74) Vertreter: Jönsson, Hans-Peter
(86) Internationale Anmeldenummer: PCT/DE2001/004401
(87) Internationale Veröffentlichungsnummer: WO 2002/041826

(56) Entgegenhaltungen:
- WO-A-97/24224
- US-A- 4 681 949
- US-A- 4 981 819
- LIU H AND TOSHIMA N: "Transferring Colloidal Metal Particles from an Organic to an Aqueous Medium and vice versa by Ligand Coordination" J. CHEM SOC. CHEM. COMM., Nr. 16, 1992, Seiten 1095-1096, XP002214903 in der Anmeldung erwähnt
- PATENT ABSTRACTS OF JAPAN vol. 2000, no. 02, 29. Februar 2000 (2000-02-29) & JP 11 319538 A (NIPPON PAINT CO LTD), 24. November 1999 (1999-11-24)

## Beschreibung

### STAND DER TECHNIK

Die vorliegende Erfindung betrifft Phasenübergänge von Kolloiden, insbesondere von Nanopartikeln eine so erhältliche Phase und ihre Verwendung, u.a bei der homogenen sowie heterogenen Katalyse mit Nanopartikeln.

Die weit verbreitete Verwendung von Kolloiden, und insbesondere sehr kleinen Kolloiden, nämlich Nanopartikeln in den Bereichen Biotechnologie, Nanotechnologie, Kolloid- und Oberflächenwissenschaften, in der Katalyse, der Elektronik, der Festkörperphysik und den Materialwissenschaften steht derzeit im Mittelpunkt von einschlägiger Forschung und Entwicklung. Der Begriff "Kolloid" wird im folgenden in synonymer Weise zu dem Begriff "Nanopartikel" verwendet, da die letzteren nur den Spezialfall sehr kleiner Kolloide bilden. Die vorliegende Erfindung lässt sich wenigstens anwenden auf Kolloide im Grössenbereich zwischen 1000 Nanometern und 0,1 Nanometern. Die jeweiligen, spezifischen Anwendungsfälle für die Verwendung der Kolloide erfordern allerdings oft spezifische Grössen, beispielsweise relativ kleine, wenn die Teilchen als Kolloid durch enge Düsen gespritzt werden sollen.

Organische, synthetische Herstellungsverfahren können in bekannter Weise Nanopartikel-Materialien von gewünschter Morphologie, Größe und Gestalt mit relativ hohen Konzentrationen herstellen, die für viele Anwendungen und für den Transport der Nanopartikel geeignet erscheinen.

Ein großer Anteil der Anwendungen dieser Partikel verlangt jedoch, daß sie in wässrigen Medien, also in wässriger Lösung, oder in Lösungen, die mit Wasser mischbar sind, also etwa Alkohole, vorliegen.

Eine Direktsynthese in Wasser führt jedoch nur zu geringen Konzentrationen der Nanopartikel, da diese sonst ausfallen. Insbesondere weist eine solche Direktsynthese Probleme auf, die zum Einen auf dem Auftreten ionischer Wechselwirkungen beruhen. Diese Probleme werden üblicherweise durch niedrige Reaktantenkonzentrationen, etwa 5 x 10⁻⁴M, überwunden. Siehe J. Turkevich, P.C. Stevensen, J. Hillier, Diskuss. Faraday Society. 1951, SS. Zum Anderen kann es schwierig sein, die für die Synthese benötigten Stabilisierungsmittel später zu entfernen. Demgegenüber lassen sich solche Partikel in organischen Lösungsmitteln bei relativ hohen Konzentrationen, bis zu 1M in Bezug auf das Edukt mit vorhersagbarer Größe und Gestalt herstellen. Dies ist beispielsweise offenbart in: M. Green, P. Obrian, Chem. Kommun. 1999, 2235 oder in: M. P. Pileni, New J. Chem. 1998, 22, 693.Auch weisen diese Partikel gegenüber jenen, die in wässrigen Lösungen synthetisiert wurden eine verbesserte Monodisperisität auf. Sie sind jedoch nicht mit Wasser mischbar, was ihre Anwendbarkeit einschränkt.

Gelöste Nanopartikel sind jedoch für viele Anwendungen notwendig, da sie in koagulierter Form neben der guten Applizierbarkeit viele von ihren positiven chemischen und physikalischen Eigenschaften verlieren.

Eine hohe Konzentration der Nanopartikel in der Lösung wird aus vielerlei Gründen je nach Anwendungsgebiet spezifisch bevorzugt. Ein genereller Vorteil einer hohen Konzentration besteht darin, daß die Lösung mit hoher Konzentration an Nanopartikeln nur ein geringes Transportgewicht aufweist im Vergleich zu einer Lösung mit einer niedrigen Konzentration.

Aus dem oben genannten Zusammenhang ergibt sich die Möglichkeit, Kolloide oder Nanopartikel in organischem Lösungsmittel zu synthetisieren und anschließend in eine wässrige oder ähnlich brauchbare Lösung überzuführen. Dabei findet ein Phasentransfer zwischen der organischen Ausgangslösung in eine im Wesentlichen anorganische Ziellösung, insbesondere eine wässrige Ziellösung statt. Ein solches Phasentransferverfahren ist für beide Richtungen offenbart in Liu, H., Toshima, N.: "Transferring Colloidal Metal Particles from an Organic To an Aqueous Medium and vice versa by Ligand Coordination", Journal of the Chemical Society, Chemical Communications, Number 16, 1992, pp.1095 to 1096.Darin ist eine technische Lehre offenbart, bei der die Nanopartikel kovalent mit Natriumdiphenylphosphinbenzolsulfonat (DPPS), einem wasserlöslichen Phosphinliganden bedeckt werden. Eine solche kovalente Bindung wird für die Zwecke der vorliegenden Anmeldung als "irreversibel" bezeichnet, da sie nur schwer wieder aufzubrechen ist.

Dieser Vorgang der kovalenten Bindung verändert jedoch die Chemie der Partikeloberfläche dauerhaft aufgrund der festen Bindung zwischen Liganden und Nanopartikel. Desweiteren sind die Verwendungsmöglichkeiten für solche, mit DPPS bedeckten Nanopartikel nur gering, da die DPPS-Moleküle als Hüllschale für viele Anwendungen nicht gewünscht sind. Die DPPS Liganden lassen sich jedoch nur mit großem Aufwand wieder von der Nanopartikeloberfläche entfernen. Ausserdem ist die in dem 1-Schritt Phasenübergang mit guter Ausbeute erhältliche, maximale Konzentration von Nanopartikeln in der Ziellösung relativ niedrig. Denn eine gute Transferausbeute ist nur mit einer festen, für das "Liu- Verfahren" optimalen DPPS Konzenztration in der wässrigen Lösung zu erreichen. Dies sind Nachteile, die für viele Anwendungen für die Nanopartikel nicht oder nur schwer in Kauf genommen werden können.

Es wäre also insbesondere wünschenswert, ähnlich hohe Konzentrationen wie bei der Synthese der Nanopartikel in organischer Lösung auch in wässriger Lösung zu haben, möglichst ohne dabei im Hinblick auf die spätere Verwendung der Nanopartikel eingeschränkt zu sein.

### BESCHREIBUNG DER ERFINDUNG

Die vorliegende Erfindung betrifft ein Phasentransferverfahren zum Überführen von anorganischen Nanopartikeln aus einer organischen Phase in eine damit nicht mischbare wässrige Phase oder alkoholische Phase unter Verwendung eines Phasentransferkatalysators, der einen Bestandteil Y, einen hydrophilen Bestandteil X und einen organischen Spacerbestandteil Z aufweist,
dadurch gekennzeichnet, dass
(i) der Bestandteil Y eine schwach basische Gruppe umfasst, der hydrophile Bestandteil X eine stark basische tertiäre Aminogruppe umfasst und diese über den organischen Spacerbestandteil Z in Konjugation zu einander stehen, oder
(ii) der Bestandteil Y eine Thiolgruppe und der hydrophile Bestandteil X eine Carboxygruppe umfasst, oder
(iii) der Phasentransferkatalysator Mercaptopropyltrimethoxysilan ist, und
eine wässrige oder alkoholische Phase, die eine Lösung von anorganischen Nanopartikeln enthält und einen Phasentransferkatalysators, der an die Oberfläche der Nanopartikel anbinden kann und der einen Bestandteil Y, einen hydrophilen Bestandteil X und einen organischen Spacerbestandteil Z aufweist, die wie unter Option (i) zuvor definiert sind.

Die Zielphase kann somit auch eine Lösung sein, die wasserlösliche Verbindungen, insbesondere Alkohole in einer Konzentration zwischen 0% und 100% enthält.

Eine idee der vorliegenden Erfindung besteht auch darin, den Phasentransferreaktant, das heisst den "Stoff" im o.g. Sinne, für den Phasentransfer gleich im Hinblick auf die spätere Verwendung der Nanopartikel auszuwählen, und ihn je nach den gewünschten chemischen Funktionen seiner Bestandteile X, Y und Z zu gestalten.

Die Zugabe des Phasentransferkatalysators bewirkt, dass die Nanopartikel ohne weiteres Zutun in einem 1-Schritt Prozess von der organischen Phase in die wässrige oder alkoholische Phase übergehen. Diese Phase wird in folgenden anhand einer wässrigen Phase erläutert.

Damit liegt eine wässrige Lösung von Nanopartikeln vor, deren Konzentrationsgrad von der Zugabe der Menge an Wasser abhängt. Das Übergehen kann durch Zuführen von Energie, beispielsweise in Form von Rühren oder Schütteln des Gemisches beschleunigt werden. Bei gleicher Menge an Wasser wie organischem Lösungsmittel liegen die gewünschten gleich hohen Konzentrationen im Wasser vor, wie sie vor dem Phasentransfer in dem organischen Lösungsmittel vorlagen.

In vorteilhafter Weise kann anschließend nach ausreichender Wartezeit eine einfache Trennung der anorganischen Phase von der wässrigen oder alkoholischen Phase vorgenommen werden. Denn die meist schwerere, wässrige oder alkoholische Phase kann leicht von der organischen Phase abgetrennt werden.

In den Unteransprüchen finden sich vorteilhafte Weiterbildungen und Verbesserungen des jeweiligen Gegenstandes der Erfindung.

Es wird für eine leichte Ablösbarkeit des Bestandteils Y der Reaktanten von den Nanopartikeln nach erfolgtem Phasentransfer vorgeschlagen, eine reversible Bindung zwischen der Nanopartikeloberfläche und den daran ankoppelnden Bestandteilen Y des Phasentranaferstoffes zu verwenden. Unter "reversibler" Bindung wird dabei im Wesentlichen eine Bindungsart verstanden, die hauptsächlich durch van der Waals Kräfte bestimmt wird. Zumindest eine starke kovalente Bindung oder eine ionische Bindung zum Nanopartikel hin soll also hierbei ausgeschlossen sein, um den Zweck leichter Entfernbarkeit der Hüllmoleküle von der Nanopartikeloberfläche 10 nicht zu gefährden.

Insbesondere wird zu diesem Zweck vorgeschlagen, das kommerziell erwerbbare 4-Dimethylaminopyridin, im Weiteren als DMAP abgekürzt, z.B. in wässriger Lösung befindlich, zu der organischen Lösung in ausreichender Menge zuzufügen. Das Pyridin stellt dabei mit seiner Ringform gleichzeitig den SpacerBestandteil Z und den Kopplungsbestandteil Y dar. Das Stickstoffatom im Ring bindet dabei an die Nanopartikeloberfläche. Es handelt sich dabei in den meisten Fällen um eine relativ schwache Bindungsart, die aber ausreicht, um die Nanopartikel in wässriger Lösung zu stabilisieren.

Die Zugabe von DMAP lediglich in einer Mindestmenge, ohne beachten zu müssen, eine Maximalmenge in Relation zur Menge der Ausgangslösung zu überschreiten, ist vorteilhaft gegenüber dem vorgenannten Stand der Technik nach Liu et al.. Die DMAP Hüllmoleküle um die Nanopartikel können nach erfolgtem Phasentransfer beispielsweise mit Toluol leicht wieder abgewaschen werden, wenn dies aus irgendeinem Grunde erforderlich sein sollte. Ein solcher Grund kann beispielsweise die Schaffung möglichst großer freier Nanopartikeloberflächen sein, die aktiv für katalytische Zwecke zur Verfügung stehen sollen, oder wenn ein elektrischer Strom mit möglichst wenig Widerstand durch eine Schicht von Metallnanopartikeln hindurch fliessen können soll. Hierbei würden die Hüllmoleküle sonst widerstandserhöhend wirken, da sich die Metallnanopartikel nicht berühren.

Soll hingegen nach erfolgtem Phasentransfer zwischen Hüllmolekülen und Nanopartikeloberfläche eine möglichst stabile Bindung bestehen, wie sie für viele Anwendungen gewünscht ist, so wird erfindungsgemäß der Stoff so ausgewählt, dass eine möglichst irreversible Bindungsart dort vorherrscht. Dies geschieht durch eine kovalente Bindung zwischen Nanopartikeloberfläche und dem Kopplungsbestandteil Y (Thiolgruppe) des Phasentransfer Katalysators (ii), bevorzugterweise Merkaptoundekan-Säure (MUA).

Wie sich aus dem vorangegangenen direkt ergibt, können nun die Herstellungsverfahren vom Stand der Technik für Nanopartikel oder Kolloide ganz allgemein, die auf organischer Synthese beruhen, durch das der Synthese nachgeschaltete Phasentransferverfahren gemäß der Erfindung unter Erzielung von vielerlei Vorteilen ergänzt werden.

Die oben genannten Phasen transfer Katalysatoren sind aus folgender chemischer Formel generisch ableitbar:

X ---- Z -----Y

Diese generische Formel enthält einen organischen Spacer Z, einen daran anbindenden hydrophilen Bestandteil X sowie einen ebenfalls an Z anbindenden Bestandteil Y, der an die Oberfläche des Kolloids oder Nanopartikels anbinden kann. 4-Dimethyl-Amino-Pyridin ist nur ein Beispiel dafür, ebenso die oben genannte, konzentrierte 11-Merkapto-Undekan-Säure (MUA)-Toluol-Lösung.

Der Bestandteil X ist von der Nanopartikeloberfläche weg gerichtet und wird gemäß Anspruch 1 gewählt werden, um für die weitere Verwendung der Nanopartikel eine spezielle Eignung zu besitzen. Beispielsweise kann er zur Ankopplung an biologisch abgeleitete Moleküle, etwa bestimmte Proteine, dienen, um mit dem Protein als Träger zu Krebszellen transportiert zu werden. Nach Anlagerung können Krebszellen dann durch gezielte Einwirkung bekämpft werden, indem physikalische oder chemische Eigenschaften der Nanopartikel gezielt ausgenutzt werden. Als Beispiel sei genannt, die Temperatur der Nanopartikel gezielt durch Bestrahlung zu erhöhen, wobei die Nanopartikel dann ihre Energie über Wärmeleitung an die Krebszellen abgeben, die dadurch ihrerseits unschädlich gemacht werden können.

Der mindestens eine Spacerbestandteil Z muss dafür geeignet sein, dass wenigstens der Bestandteil Y und der Bestandteil X (16) daran anbinden können, und er muss eine molekulare Ausdehnung besitzen, die ausreichend groß ist, damit die Bestandteile Y und X ihre chemischen Wirkungen aufgrund ihrer jeweiligen chemischen Affinitäten entfalten können. Gegebenenfalls kann eine mehrgliedrige Verbindung, etwa eine Ringverbindung dabei auch die Aufgaben von zweien der drei Bestandteilen übernehmen.

Damit können die jüngsten Fortschritte des Standes der Technik, praktisch alle relevanten Stoffe als Nanopartikel in organischer Phase zu synthetisieren, nun noch unmittelbarer einer wirtschaftlichen Verwertung zugeführt werden. Denn sie können in sehr hoher Konzentration in Wasserlösung mit dem Phasen transfer Katalysator als Stabilisator gehalten werden. Die Konzentration kann auch erhöht werden, indem ein Teil des Wassers beispielsweise abdestilliert wird, oder der Phasentransfer Katalysator in Lösung mit nur relativ wenig Wasser zugeführt wird.

Der Bestandteil X weist hydrophile Eigenschaften aufweist, mit einer Affinität zu Wasser, die ausreichend groß ist, um den Phasentransfer zu bewirken.

Damit können anorganische Kolloide, insbesondere Nanopartikel mit Durchmesser in einer Dimension zwischen 0,1 nm und 1000nm oder bei Kolloiden grösser, die durch Reaktionen anorganischer Salze oder einer Mischung aus anorganischen Salzen in nichtwässrigen Lösungsmitteln in wässrige oder alkoholische Lösungen dadurch transferiert werden, dass der Phasentransfer Katalysator in ausreichender Menge adsorbiert wird.

Die Verwendung eines Thiols als Bestandteil Y und einer Carbonsäure als Bestandteil X in dem erfindungsgemäß verwendeten Phasentransfer katalysator ist eine Ausführungsform der Erfindung.

Wenn der Bestandteil Y so gewählt ist, dass er eine kovalente Bindung an die Kolloidoberfläche bewirkt, etwa wie im Falle der Verwendung von MUA mit aliphatischer Verbindung so wird eine stabile, permanente Bindung erreicht, die für viele Anwendungen gewünscht ist, beispielsweise bei der Herstellung von Farben, Tinten, etc..

Wenn der Bestandteil Y eine nicht-kovalente Bindung an die Nanopartikeloberfläche bewirkt, ergibt sich der Vorteil, dass die Oberfläche der Nanopartikel nur temporär und nicht permanent modifiziert wird. Dies kann beispielsweise durch Verwendung von DMAP mit konjugierter Bindung geschehen. Durch Waschen kann die Nanopartikeloberfläche nach erfolgtem Phasenübergang wieder freigelegt werden. Daher können beispielsweise bei Verwendung metallischer Nanopartikel benachbarte Nanopartikel elektrischen Strom leiten. Desweiteren wird eine gewaschene Oberfläche der Nanopartikel beispielsweise bei der Katalyse vorteilhaft ausgenutzt, um deren Effizienz zu steigern.

Die chemische Wirkung dieser besonderen erfinderischen Massnahme bewirkt ein Aufbringen des Phasentransfer Katalysators auf die Oberfläche von Nanopartikeln, z.B., Metall- oder Edelmetallnanopartikel, Gold, Silber, Iridium, Platin, Palladium, ohne deren Oberfläche durch eine kovalente Bindung zu modifizieren, wie es im Stand der Technik der Fall ist, etwa bei Bildung einer 'shell' aus Goldsulfid - als Hülle um einen Nanopartikel herum. Das erfindungsgemäß verwendeten Molekül enthält somit im wesentlichen einen hydrophilen Teil, der gerne an Wasser ankoppelt, sowie einen weiteren Teil; der an das Nanopartikel ankoppelt, sowie einen dazwischen liegenden Abstandhalter, 'Spacer' genannt. Das oben genannte DMAP ist dafür ein Beispiel.

Gemäß seinem breitesten Aspekt stellt sich das erfindungsgmässe Phasentransferverfahren für Kolloide und insbesondere Nanopartikel wie folgt dar:

Die Erfindung bretrifft somit ein Verfahren zum Transferieren von Kolloiden, insbesondere von Nanopartikeln aus einer organischen Ausgangslösung in eine Ziellösung, wobei die Ziellösung entweder eine wässrige Lösung oder eine Lösung ist, die wasserlösliche Verbindungen, insbesondere Alkohole in einer Konzentration zwischen 0% und 100% enthält. Es ist gekennzeichnet durch die Schritte:
a.) Zufügen eines vorgewählten Stoffes nach einem der Ansprüche in die organische Ausgangslösung in ausreichender Menge,
b.) Übergehen-Lassen der Nanopartikel von der organischen Phase in die wässrige oder alkoholische Phase,
c.) Trennen der wässrigen oder alkoholischen Phase von der organischen Phase. Damit werden hohe Nanopartikelkonzentrationen in der Ziellösung gewonnen, wobei die Konzentration abhängig ist von der Menge an Ziellösung, die vorher vorhanden oder zur Ausgangslösung zugegeben wird.

Wenn ein erfindungsgemäß verwendeter Phasentransfer Katalysator, also etwa DMAP oder MUA gelöst in wässriger Lösung oder in einer, wasserlösliche Verbindungen, insbesondere Alkohole in einer Konzentration zwischen 0% und 100% enthaltenden Lösung mit vorgegebener Konzentration darin zugefügt wird, kann das Verfahren an die jeweilig vorhandenen Anforderungen bei der Produktion angepasst werden. Insbesondere können wässrige Lösungen mit sehr hoher Konzentration von Nanopartikeln hergestellt werden.

Wenn eine Menge des Phasentransfer Katalysators zugeführt wird, die groß genug ist, um eine Monoschicht um einen jeweiligen, in der Lösung vorkommenden Nanopartikel zu bilden, so ergibt sich insbesondere bei MUA eine grosse Stabilität der Nanopartikel.

Das Verhältnis der Anzahl der Oberflächenatome eines Nanopartikels zur Anzahl des daran anbindenden Phasentransfer Katalysators kann bevorzugt in einem Bereich zwischen 0,1 und 10, weiter bevorzugt um 1 herum liegen.

Es können dabei metallische Kolloide, insbesondere metallische Nanopartikel und auch legierte (alloys) Kolloide oder Nanopartikel transferiert werden.

Der Transfer von Nanopartikeln aus Gold, Silber, Iridium, Platin, Palladium, Nickel, Eisen, Rhodium, Ruthenium oder Metalloxide, insbesondere Eisenoxid,- Zinkoxid, Titandioxid, Zinnoxid, ergibt dabei jeweils gewünschte Effekte, beispielsweise langzeitstabile Farbstoffe oder Beschichtungen mit anderen gewünschten physikalischen oder chemischen Eigenschaften, etwa elektrische, magnetische oder andere.

Auch Halbleiter-Nanopartikel sowie anorganische Nanopartikel können transferiert werden, die Elemente aus den Seltenen Erden enthalten.

### Der zusätzliche Schritt:

Trennen der Kolloide, insbesondere Nanopartikel von dem Lösungsmittel der Ziellösung zum Erhalt von Pulver oder Breisubstanz aus Partikeln, insbesondere von Nanopartikeln, ermöglicht eine weiteren Aggregatzustand der Nanopartikel, der alternativ zur flüssigen Form der Ziellösung für diverse Anwendungsfälle der Weiterverarbeitung bevorzugt sein kann.

In vorteilhafter Weise wird der Phasentransfer Katalysator (PTK) in einer solchen Menge zugeführt, die groß genug ist, um eine Monoschicht um einen jeweiligen, in der Lösung vorkommenden Nanopartikel zu bilden. Diese Schicht enthält also soviele PTK-Moleküle, um den Nanopartikel zu bedecken. Eine zu hohe Dosierung von PTK schadet jedoch nicht im Sinne der Erfindung. Wenn Goldnanopartikel verwendet werden, so liegt das Verhältnis der Anzahl der Oberflächenatome eines Nanopartikels zur Anzahl des daran anbindenden PTKs bevorzugt in einem Bereich zwischen 0,1 und 10, weiter bevorzugt um 1 herum.

Hohe Konzentrationen von Edelmetallnanopartikeln, Edelmetallkolloiden können erfindungsgemäß nun in Wasser gelöst verwendet werden, und zwar in einer Form, die speziell anpassbar ist für die spätere Verwendung der Nanopartikel. Damit werden Transportkosten gesenkt, denn die realisierbaren Konzentrationen können um den Faktor 10⁶ bis 10⁹ (zehn hoch sechs bis zehn hoch neun) gesteigert werden, im Vergleich zu heutigen Konzentrationen in Wasser. Damit sinkt das Transportgewicht einer die Nanopartikel enthaltenen Lösung um eben diesen Faktor bei gleicher chemischer Wirksamkeit. Unter 'hoher' Konzentration sollen jedoch auch Konzentrationen verstanden werden, die weniger als 10⁶ mal höher sind als die heute erhältlichen Konzentrationen in Wasser, wie sie beispielsweise heute kommerziell angeboten werden.

Damit sind farbechte, langzeitstabile Farbsubstanzen auf Wasserbasis herstellbar. Lippenstifte, KFZ-Lackfarben, sogar Druckfarben können von diesen Eigenschaften auf extreme Weise profitieren.

Denn solche Farben werden erfindungsgemäß auch druckbar, weil die erfindungsgemäßen Farbsubstanzen aufgrund der geringen Größe der farbgebenden Nanopartikel keine feinen Düsen einer Druckmaschine, z.B. Tintenstrahldrucker, mehr verstopfen. Ein erfindungsgemäßer Farblack, bzw. Eine Farbschicht ist im Vergleich zu Farben mit größeren Microfarbpartikeln vom Stande der Technik viel feiner von der Oberflächen- und seiner inneren Struktur her. Damit eignet sich jedoch eine Farbe erst für viele Anwendungsbereiche, da die Farbschicht aufgrund ihrer homogenen Struktur nicht mehr so leicht wegblättert.

Mindestens die folgenden Verwendungen der Ziellösung oder Pulvers bzw. Breisubstanz sind von der vorliegenden Erfindung mitumfasst:
Zum selektiven Beschichten von Oberflächen makroskopischer Körper, oder als Farbe, insbesondere als Druckfarbe, oder Lack, oder zum Anhängen an biologisch abgeleitete Moleküle, insbesondere als Biomarker;
zur Herstellung von auf einen Trägerkörper aufgebrachten Strukturen mit vorbestimmten magnetischen Eigenschaften;
zur Herstellung von auf einen Trägerkörper aufgebrachten Strukturen mit vorbestimmten elektrischen Eigenschaften;
in der Sol/Gel- Verarbeitung; oder die
   Verwendung einer Ziellösung, um Trägerpartikel mit wenigstens einer Schicht aus Kolloiden, insbesondere Nanopartikeln zu beschichten, wobei eine dafür hinreichende Affinität zwischen Trägerpartikel und Kolloid oder Nanopartikeln besteht. Dabei kann der Verfahrensschritt des Mischens einer die Trägerpartikel enthaltenden Trägerlösung mit der Ziellösung erfolgen. Oder die Trägerpartikel können in anderer Form in die Ziellösung eingemischt werden.

Dabei kann der zusätzliche Schritt durchgeführt werden, Reste des Phasentranfer Katalysators durch Waschen mit einem geeigneten (organischen) Lösungsmittel zu entfernen.

Für viele Verwendungen der erfindungsgemässen, wässrigen Lösungen, beispielsweise für Lackierungen im Kraftfahrzeugbereich, kann eine wässrige Lösung als solche oder in Mischung mit einer anderen Komponente auch zunächst auf kleine Trägerpartikel (beads), beispielsweise durch Sprühen per Inkjetverfahren nach dem Stand der Technik, aufgebracht werden, um in einem späteren Schritt als Komponente für eine Lackierung einer oder mehreren anderen Komponente beigefügt zu werden, und zusammen mit diesen und in homogener Verteilung darin auf den zu lackierenden Gegenstand in üblicher Weise aufgebracht werden. Das Aufsprühen von beads als solches ist im Stand der Technik bekannt, etwa bei einer Perleffekt- oder Metalliklackierung.

Die obengenannten Trägerpartikel (beads) stellen aufgrund ihrer guten Handhabbarkeit - versehen mit Nanopartikeln aus wässriger, hochkonzentrierter Lösung einen eigenständig verkehrsfähigen Schutzgegenstand dar. Ihre Grösse ist prinzipiell abhängig vom jeweils gewählten Farbauftrageverfahren des Standes der Technik. Solche Trägerpartikel können dann je nach der gewünschten Funktion der Nanopartikeleigenschaften industriell eingesetzt werden.

In besonders bevorzugter Weise können funktionalisiert umhüllte Nanopartikel, auf besonders kleine Trägerkörper aufgebracht werden, mit einer Größe von 0,02 Mikrometer oder grösser. Je nach gewünschter weiterer Verwendung des Systems aus Trägerkörpern und Nanopartikeln darauf ist es dann gewünscht, die Hülle der Nanopartikel darauf zu belassen (dann sollte z.B. MUA verwendet werden) oder relativ leicht entfernen zu können (entsprechend DMAP), beispielsweise durch Waschen mit Toluol.

Besonders geeignet erscheinen dabei Edelmetall-, insbesondere Gold (Au)- Nanopartikel mit einer DMAP-Hülle, wenn sie etwa auf polyelektrolytbeschichteten Kügelchen aus Polystren, Polymethylmethacrylat (PMMA), oder einem Siliziumoxid, etc. aufgebracht, und danach mit einer Lösung enthaltend Hydroxylaminhydrochlorid und Wasserstofftetrachloraurat behandelt werden (electroless plating), um durch Entfernen der reversibel gebundenen Phasentransfer Katalysators - z.B., DMAP oder Resten davon eine zusammenhängende Metallhülle, bzw. Goldhülle auf den Kernen der Kügelchen zu bilden. Solche Kügelchen/ Oberflächen können dann vorteilhaft in einem weiten Bereich industrieller oder medizinischer Anwendungen Verwendung finden, etwa im Bereich Photonik, Krebstherapie, Galenik und Katalyse, wie weiter oben erwähnt. Gemäss der vorliegenden Erfindung können in einem einzigen Adsorptionsschritt Kügelchen mit hoher Beladung an Nanopartikeln hergestellt werden, wobei in hohem Maße homogene Hüllsphären erhalten werden. Auch hier werden durch die Erfindung erhebliche Vorteile erzielt, die jeweils spezifisch sind je nach Anwendung.

Einen weiteren eigenen, verkehrsfähigen Gegenstand bilden Farbflüssigkeiten, in die Nanopartikel aus wässriger Lösung gemäß der Erfindung eingebracht worden sind.

Desweiteren kann in vorteilhafter Weise erfindungsgemäß ein Grundfarbenset wässriger Lösungen zur Herstellung von Mischfarben zur Verfügung gestellt werden, wobei in bevorzugter Weise Goldnanopartikel zur Herstellung der Grundfarbe Rot, Silbernanopartikel zur Herstellung der Grundfarbe Gelb und Iridiumnanopartikel zur Herstellung der Grundfarbe Blau verwendet werden. Bei entsprechender Dosierung der Grundfarben können daher alle Mischfarben wie üblich hergestellt werden, z.B. die Mischfarbe Grün durch Vermischen von wässrigen Lösungen oder auch beads mit Iridiumnanopartikeln (blau) und Silbernanopartikeln (gelb).

Wenn in dem erfindungsgemäßen Verfahren metallische Nanopartikel transferiert werden, so ergeben sich aufgrund der breiten Palette an Verfahrensendprodukten hochkonzentrierte Nanopartikellösungen mit den jeweils gewünschten Metallen. Wenn beispielsweise eine Eisen- und eine Platinlösung hergestellt werden, so kann ein Beitrag zur Herstellung spritzbarer Flüssigkeiten zur Herstellung von auf einen Trägerkörper gespritzten, gesprühten oder anders aufgetragenen magnetisierbaren oder permanentmagnetischen oder elektrisch wirksame Strukturen, etwa Leiterbahnen, geleistet werden, mit dem beispielsweise sehr klein strukturierte Speichermedien "gedruckt" werden können, die dann in bekannter Weise durch einen Induktionskopf geeigneter kleiner Grösse gelesen oder geschrieben werden können.

Gemäß einem zweiten Hauptaspekt der vorliegenden Erfindung ergibt sich als unmittelbare Anwendung des erfinderischen Prinzips, eine Katalyse durchzuführen, und zwar entweder eine homogene oder heterogene Katalyse, die in den üblichen Bereichen, z.B. Polymerherstellung, etc. anwendbar ist, und damit für viele industriell durchgeführte chemische Prozesse und im Alltag vorkommende Prozesse von immenser Wichtigkeit ist:

Gemäß diesem Aspekt wirken als Katalysatorstoff hier z.B. Edelmetallnanopartikel, die beispielsweise in organischer Lösung befindlich einem organischen Reaktionsgemisch beigegeben werden. Sie verteilen sich gleichmäßig - homogen - in der Reaktionsflüssigkeit und sind nicht an Trägermoleküle, wie etwa Zeolite gebunden, wodurch sie eine katalytische Effizienz entwickeln, die um ein Vielfaches höher ist als bei der heterogenen Katalyse mit entsprechender Bindung an ein Träger, wie etwa Ceolite, Kohle, etc.

Nun kann die Grundreaktion durchgeführt werden, wobei die katalytische Wirkung vorteilhaft zum Tragen kommt, weil nun ein Vielfaches der Oberfläche - 200 qm pro Gramm bei 6 Nanometer großen Nanopartikeln- katalytisch wirksam ist im Verhältnis zu heterogenen Katalysatoren, bei denen ein Großteil des Katalysators chemisch inaktiv bleiben muß, weil er im Innern des Trägermoleküls versteckt bleibt.

Nach erfolgter Reaktion soll der Katalysatorstoff in vorteilhafter Weise wieder aus dem Reaktionsgemisch entfernt werden können. Dies geschieht nun dadurch, dass Wasser und ein erfindungsgemäßer Stoff hinzugefügt wird. Die Nanopartikel gehen dann wieder wie oben beschrieben von der organischen Phase in das Wasser über, und können somit vorteilhaft vollständig wiedergewonnen werden. Gleichzeitig wird das Reaktionsgemisch in vorteilhafter Weise frei vom Katalysatorstoff.

Auch die Belegung eines für ein heterogenes Katalyseverfahren geeigneten Substrates wie Zeolite oder Kohle mit Nanopartikeln mit einer gegebenenfalls erfinderisch gewonnenen, wässrigen Lösung mit hoher Konzentration von Nanopartikeln, Z.B. Gold-, oder Platin-Nanopartikeln, kann erfindungsgemäß dadurch verbessert werden, dass die Nanopartikel durch Zugabe der erfindungsgemäßen, wässrigen Lösung einfacher, beispielsweise ohne Verdampfen der Nanopartikel an die meist hydrophilen Trägerstoffe gelangen können. Dies erleichtert und verbilligt entsprechende, industriell verwendete Katalysatoren.

Das erfindungsgemäße Phasentransferverfahren ist anwendbar auf alle Nanopartikel, die in der gleichzeitig anhängigen Patentanmeldung PCT/DE-00/03130 offenbart sind. Insoweit kann das hier vorliegende Verfahren in vorteilhafter Weise mit der Offenbarung der genannten Anmeldung kombiniert werden, woraus sich die für den Fachmann bekannten Vorteile ergeben.

### ZEICHNUNGEN

Ausführungsbeispiele der Erfindung sind in den Zeichnungen dargestellt und in der nachfolgenden Beschreibung näher erläutert.

Es zeigen:
- Fig. 1: eine schematische Abbildung eines Nanopartikels mit angekoppelten MM- Molekülen, mit beispielhafter Strukturformel (DMAP);
- Fig. 2: eine Photografie der Goldnanopartikel in einer 2-Phasen Mischung vor (rechts) und nach (links) dem Transfer innerhalb 2mL Eppendorf Röhrchen;
- Fig. 3: ein TEM Bild der Goldnanopartikel aus Fig. 1, nachdem sie in Wasser transferiert wurden;
- Fig. 4: Photographien 5 verschiedener Nanopartikelproben paarweise (A,B) (B,C), ... jeweils vor und nach dem Phasentransfer unter Verwendung von MUA als MM,
- Fig. 5: UV/VIS- Spektren einer von Gold -Nanopartikeln in Toluol (durchgezogene Linie) und nach dem Transfer (gestrichelt),
- Fig. 6: TEM-Aufnahmen von Gold-Nanopartikeln nach der Herstellung in Toluol (A) und 1 Monat nach dem Transfer,
- Fig. 7: TEM-Aufnahmen von Palladium Nanopartikeln in Toluen (A) und nach dem Transfer in Wasser (B), bewirkt durch DMAP als M,
- Fig. 8: EDAX- Spektren von Palladium Nanopartikeln wie synthetisiert (oben) und nach dem Pasentransfer in Wasser (unten),
- Fig. 9: Skizze eines Mechanismus (Schema 1) für den Phasentransfer von Gold- und Palladiumnanopartikeln aus Toluol in Wasser durch Zugabe von DMAP, R = C8H17,
- Fig. 10: Photographien, die den Zeitverlauf beim Phasentransfer zeigen, Goldnanopartikel aus Toluen in 0.1M DMAP Lösung;

### BESCHREIBUNG DER AUSFÜHRUNGSBEISPIELE

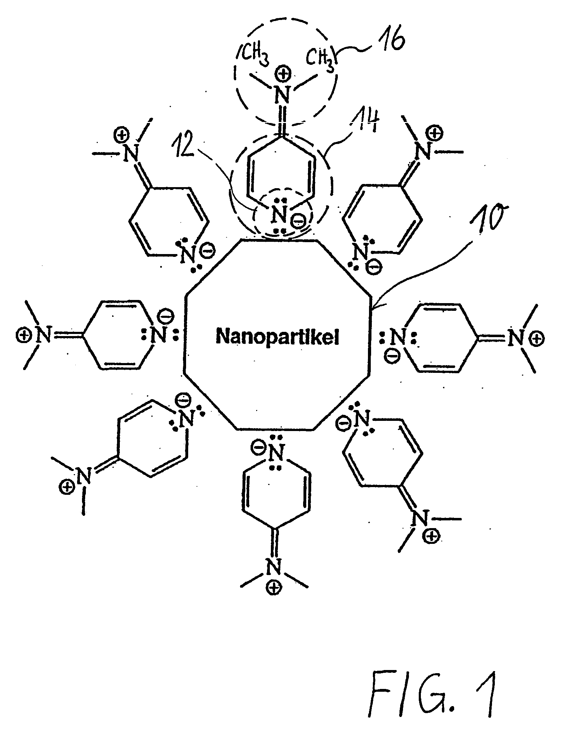

Fig. 1 zeigt im mittleren Bereich die Oberfläche 10 eines Goldnanopartikels schematisch. Die Oberfläche ist als Linie mit glatter, beispielhaft gewählter und nur schematisch zu verstehender, achteckförmiger Kontur gezeichnet. Die Oberfläche ist idealisiert glatt dargestellt, trägt jedoch einzelne Goldatome.

An seine Oberfläche 10 ankoppelnd sind schematisch acht DMAP-Moleküle gezeichnet, von denen der Einfachheit nur eines (das oberste) näher beschrieben ist. Je nach Größe der phasentransferierten Nanopartikel sind natürlich erheblich mehr Hüllmoleküle in der Zeichenebene angeordnet. Für die anderen DMAP Hüllmoleküle, die an den Nanopartikel anbinden (auch die ausserhalb der Zeichenebene liegenden) soll die Beschreibung ebenfalls gelten:

Das endozyklische Stickstoff (N) -Atom 12 des Pyridinrings bindet als Bestandteil Y des erfindungsgemäß verwendeten DMAP - Moleküls an die Nanopartikeloberfläche 10. Der Pyridinring 14 selbst dient aber auch als Abstandhalter oder Spacer Z zu dem oben abgebildeten, hydrophilen Dimethylamin 16, dem Bestandteil X des MM-Moleküls.

Die chemische Wirkung besteht nun im Aufbringen der DMAP Moleküle auf die Oberfläche von Nanopartikeln, so dass zumindest ein großer Teil der Oberfläche des Nanopartikels mit solchen Molekülen besetzt ist, im Sinne einer Monolayer mit nicht zu großen Lücken. Sollten keine Lücken vorhanden sein oder ein Überangebot an DMAP-Molekülen präsent sein, so schadet dies nicht für die gewünschte Wirkung.

Gemäß Ausführungsbeispiel wird also eine leicht entfernbare Schicht um jedes einzelne der Nanopartikel gebildet, ohne deren Oberfläche zu modifizieren, wie es im Stand der Technik der Fall ist, etwa bei Bildung einer 'shell' aus Goldsulfid - als Hülle um ein Nanopartikel herum.

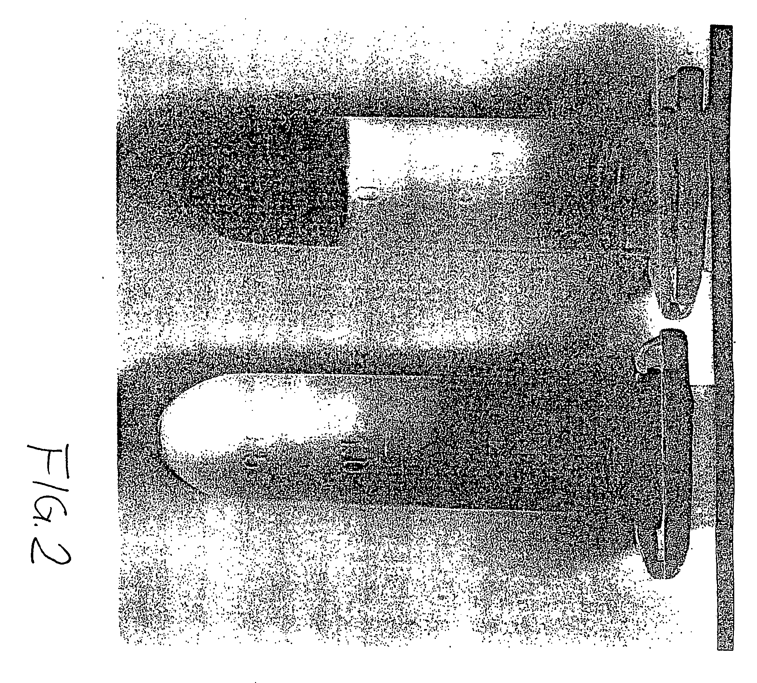

Fig. 2 zeigt eine Photografie der Goldnanopartikel in einer 2-Phasen Mischung vor (rechts) und nach (links) dem Transfer innerhalb 2mL Eppendorf Röhrchen. Die obere Phase ist Toluol, die untere Wasser. Aus der Abbildung geht klar hervor, dass keine Agglomeration von Nanoteilchen stattgefunden hat, weder in der organischen noch in der anorganischen Phase.

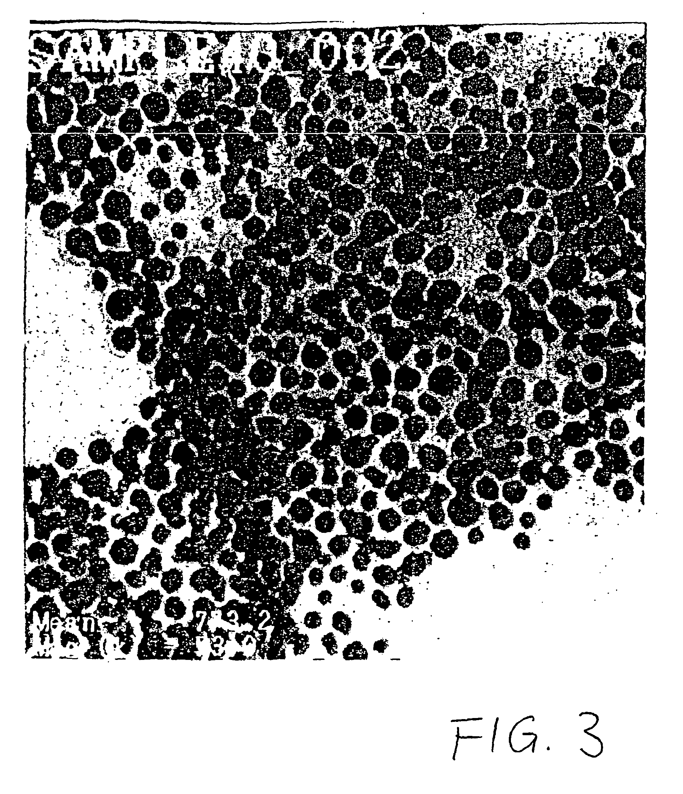

Fig. 3 zeigt ein TEM Bild der Goldnanopartikel gemäß Fig. 2, nachdem sie in Wasser transferiert wurden. Auch hieraus geht klar hervor, dass weder eine Agglomeration noch eine Änderung der Größe, Gestalt/ Form oder Morphologie der Nanopartikel während des Phasentransfers stattgefunden hat.

Selbst 6 Monate nach dem Phasentransfer weisen die transferierten Partikel keine Anzeichen von Zersetzung oder Aggregation auf. Daher ist anzunehmen, daß sie beliebig lange stabil sind. In vorteilhafter Weise benötigt dieses Verfahren weder Niederschlagsbildung noch Lösungsmittelaustausch, und die mit ihm transferierten Partikel werden auch nicht durch kovalent gebundene Liganden stabilisiert. Gerade im Hinblick auf künftige Anwendungen ist dies ein wichtiger Unterschied zu Thiolstabilisierten Partikeln. Die Verfügbarkeit solch konzentrierter, wässriger Lösungen von Nanopartikeln eröffnet neue Möglichkeiten für das Cyto-Labelling, die heterogene und die homogene Katalyse, die Festkörperphysik und für Anwendungen im Bereich kolloidaler Kristalle.

Außerdem kann grundsätzlich eine Beschichtung von Oberflächen vorgenommen werden, mit dem Zweck, die Oberfläche so zu verändern, daß sie bestimmte, gewünschte Eigenschaften der Nanopartikel oder Eigenschaften von Elementen, die in den Nanopartikeln enthalten sind, von diesen übernimmt.

Weitere Anwendungen ergeben sich im Bereich von homogener oder heterogener Katalyse, wie weiter hinten ausgeführt wird. Da die Nanopartikel auf Grund ihres Vorliegens feinverteilt in wässriger Lösung oder in beispielsweise mit Alkohol angereicherter Lösung industriell gut verarbeitbar sind, können sie beispielsweise auch auf kleine Trägerpartikel, die sogenannten Beads aufgebracht werden. Geeignet sind Trägerpartikel im Bereich etwa zwischen der doppelten Größe der verwendeten Nanopartikel (bei homogener Größenverteilung), also etwa ab einem Nanometer bis in den makroskopischen Bereich hinein, also etwa bis zu mehreren Millimetern Größe.

Hier erschließen sich industrielle Anwendungen im Bereich kolloidalen Kristallanwendungen (colloidal crystal applications, photonics), im Bereich der Lichtleitung für die Telekommunikation, um gewünschte, optische Gittereigenschaften herzustellen.

Im Folgenden werden die Herstellungsschritte geschildert:

Alle für die folgend beschriebenen Ausführungsbeispiele notwendigen chemischen Ausgangsstoffe wurden verwendet, wie sie von der Firma SIGMA-Aldrich kommerziell erwerblich sind. Das verwendete Wasser wurde vor seiner Verwendung durch ein MILLIPORE Reinigungssystem geschickt, wonach es einen Widerstand größer als 18 M Q cm besitzt. UV/VIS-Proben werden in Quarzkristall-Küvetten (Hellmar, SUPRASIL, Weglänge 1,000 cm) gebracht und unter Verwendung eines Doppelstrahl-Spectro-Photometers (CARY 4E, Varian) analysiert.

Ein Lösungsmittelspektrum wird von allen Spektren subtrahiert. Proben, die eine Sedimentation benötigen, werden in zwei Milliliter großen, stellbaren Eppendorf-Röhren zentrifugiert (3K30, SIGMA Laboratory Zentrifuges). Proben für die TEM-Messung (Phillips CM70, 120 kV Beschleunigungsspannung) werden auf Kupfergittern, die mit einer Standard-Kohleschicht versehen sind, aufgebracht und vor der Untersuchung getrocknet. Die Partikelgrößenverteilung wird mit Hilfe von Ultrazentrifugierung unter Verwendung von Absorbtionsoptik (Beckmann Optima XL-I) oder durch Analyse von TEM-Mikroskopie durchgeführt. Proben für die TEM-Analyse werden präpariert, indem ein Tropfen der Lösung auf kohlebeschichteten Kupfergittern luftgetrocknet wird.

Die Partikel werden synthetisiert, wie es veröffentlicht ist in: Hayat, M.A. (Academic PressInc., San Diego, USA, 1989), oder in Goia, D.V. & Matijevic, E. Tailoring the particle size of monodispersed colloidal gold. Colloids Surf. A 146, 139-152 (1999), oder in
Green, M. & O'Brien, P. recent advances in the preparation of semiconductors as isolated nanometric particles: new routes to quantum dots. Chem. Commun., 2235-2241 (1999).
Insoweit kann im Zweifelsfall auf die Offenbarung in diesen Quellen zurückgegriffen werden.
1)
   Der Menge von einem Milliliter eines Aliquots der somit hergestellten Nanopartikelgemische, beispielsweise mit Gold, Silber, Iridium, Platin, Palladium, Rhodium und Ruthenium, synthetisiert in Toluol und stabilisiert durch die Tetra-Alkyl-Ammonium-Salz-Verfahren wird nun erfindungsgemäß ein Milliliter einer wässrigen 4-Dimethyl-Aminopyridin-Lösung (DMAP) zugefügt. Größere Volumina von Nanopartikeln in ihrer Reaktionslösung können ebenso erfolgreich in einen Milliliter Wasser transferiert werden, wodurch ein anschließendes Recycling des Ammonium-Salzes möglich wird. Der direkte Phasenübergang über die organisch/wässrige Grenze hinweg wird innerhalb von einer Stunde bis drei Stunden ohne weiteres Zutun vollzogen. Ein schnellerer Phasenübergang kann beispielsweise durch die Verwendung von Zentrifugierungsmaßnahmen, Schütteln, Rühren, also Zufuhr von Energie erreicht werden. Daraus ergeben sich hohe Konzentrationen der Nanopartikel, die für nachfolgende Verwendung (Analytik, Photografie) noch etwa 1000-fach verdünnt werden können.
2)
   Zu einem 1-Milliliter-Aliquot der Nanopartikelmischung, wie sie oben anfangs beschrieben wurde, also etwa mit Gold, Silber, Platin, Iridium, etc. und synthetisiert in Toluol und stabilisiert durch die Tetra-Alkyl-Ammonium-Salz-Verfahren oder synthetisiert durch das Wilcoxon-AOT-Verfahren, wie es im US-Patent 5,147,841 offenbart ist, wobei keine vorhergehende Reinigung erforderlich ist, wird 100 Microliter einer konzentrierten 11-Merkaptoundekan-Säure (MUA)-Toluol-Lösung zugefügt. Die Adsorbtion des Phasentransfer-Katalysators kann mit bloßen Augen als eine rote Verschiebung in der Lösungsfarbe, gefolgt von einer einsetzenden Trübung, die durch Partikel-Agglomeration und Ausfällung erzeugt wird, wahrgenommen werden. Beschichtete Partikel können entweder aus der organischen Lösung vorsichtig zentrifugiert werden oder über Nacht der Sedimentation überlassen bleiben. Ein Waschvorgang des Präzipitats mit 2 Aliquots der Anfangslösung, gefolgt von einem Aliquot mit Methanol entfernt alle Reaktionsnebenprodukte und den Überschuß an Phasentransfer-Katalysator. Ein Waschvorgang mit Methanol schließt sich an.
   Ein vorsichtiges Schütteln des Präzipitats in basischem Wasser resultiert in einer stabilen, klaren Lösung der Nanopartikel. Da die Partikel dem Wasser als Festkörper zugefügt werden, können Lösungen beliebiger Konzentration erzeugt werden.
   Ein mit MUA durchgeführter Phasentransfer bringt ein stabil lagerbares Präzipitat hervor, das beispielsweise als Pulver oder Brei für lange Zeit stabil gehalten werden kann. Dies gilt insbesondere dann, wenn MUA-moleküle die Nanopartikel so vollständig umhüllen, dass diese nicht miteinander in Berührung kommen können. Daher ist keine Agglomeration oder Verklumpung möglich.
3)
   Einem 1-Milliliter-Aliquot der Gold-Nanopartikelmischung, wie sie oben beschrieben herstellbar ist, synthetisiert in Toluol oder synthetisiert durch das Wilcoxon-AOT-Verfahren, US-Patent 5,147,841, ohne vorhergehendes Reinigen, werden 10 Microliter von Mercapto-Propyl-Trimetoxysilan zugefügt. Die Adsorbtion des Phasentransfer-Katalysators kann mit den bloßen Augen als eine Rotverschiebung der Lösungsfarbe gefolgt von einsetzender Trübung, verursacht durch Partikel-Agglomeration und Präzipitation wahrgenommen werden. Beschichtete Partikel können entweder vorsichtig aus der organischen Lösung abzentrifugiert werden oder können über Nacht sedimentiert werden. Ein Waschvorgang des Präzipitats mit zwei Aliquots der Anfangslösung gefolgt von einem Aliquot Methanol entfernt alle Reaktionsnebenprodukte und einen Überschuß an Phasentransfer-Katalysator.

Der Phasentransferkatalysator wird zu der organischen Nanopartikellösung bei einer Metallkonzentration zwischen 1 x 10-6 bis 100 Gew.%, insbesondere 1 mg pro mL Metall in einer Konzentration zwischen 1 x 10-6 bis 100 Gew.%, insbesondere gleiche Volumina einer 0,01M wässrigen Lösung gegeben. Dabei ereignete sich ein vollständiger Phasentransfer von der organischen Lösung in Wasser.

Wie aus der vorangegangenen Beschreibung ersichtlich, kann der erfindungsgemäß offenbarte Phasenübergang der Nanopartikel aus organischer Lösung in wässrige Lösung durch die obigen zwei Ausführungsbeispiele erreicht werden. Auch andere Substanzen sind dafür möglich, wenn sie die erforderlichen Bindungseigenschaften aufweisen. Siehe dazu auch oben, Beschreibung zu Fig. 1.

Das erfindungsgemäße Phasentransfer-Verfahren kann in vorteilhafter Weise für metallische Nanopartikel wie Gold, Silber, Iridium, Platin, Palladium, Nickel, Eisen, Metalloxid, insbesondere Eisenoxid, Zinkoxid, Titandioxid und Zinnoxid Nanopartikel sowie für Rhodium- und Rhutenium-Nanopartikel verwendet werden. In vorteilhafter Weise lassen sich damit langzeitstabile Farbstoffe gewinnen, die in wässriger Lösung vorliegen. Diese können somit auch für Anwendungen herangezogen werden, bei denen sich normalerweise eine Verwendung solcher Nanopartikel in organischer Lösung aus Umweltschutzgründen, oder gesundheitlichen Gründen oder anderen Gründen verbietet. Ebenso kann das Verfahren, wie es oben beschrieben wurde, für die Markierung von Stoffen in der Elektronen-Mikroskopie verwendet werden.

Ebenso können Halbleiter-Nanopartikel transferiert werden.

Es lassen sich folgende Anwendungen und Vorteile erzielen:

Biologische Anwendungen, insbesondere Bio-Labelling, Gewinnen von Leuchteffekten durch Fluoreszenz, und andere Anwendungen, die in der oben genannten gleichzeitig anhängenden Patentanmeldung offenbart sind,

alle umweltschutzbezogenen Anwendungen, die von der Lösbarkeit der Nanopartikel in Wasser anstelle von Organik profitieren, unabhängig vom Typ der Nanopartikel.

Desweiteren kann eine anorganische, insbesondere wässrige Lösung, wie sie durch das erfindungsgemäße Verfahren gewonnen wird, daher in vielfacher Weise eingesetzt und wirtschaftlich ausgenutzt werden. Sie kann insbesondere auch als Farbe, Farbkomponente, Druckfarbe oder Lack oder als Bestandteil einer Lackierungsschicht verwendet werden. Dabei kann von der besonderen Feinheit der Nanopartikel und ihrer engen Größenverteilung profitiert werden, in einem Fall, bei dem eine Nanopartikel enthaltende Farbe durch die feinen Düsen eines Tintenstrahldruckers gebracht werden müssen. Erfindungsgemäß verstopfen dann solche Düsen nicht.

Wenn eine solche Nanopartikel enthaltende Farbe oder ein entsprechender Lack anders als durch Durchströmen einer Düse auf ein Substrat aufgebracht wird, dann ergibt sich aufgrund der engen Größenverteilung und der geringen Größe der Nanopartikel eine Lackschicht, oder Farbschicht, die weniger brüchig ist, als bei Verwendung von farbgebenden Partikeln mit Partikelgrößen im Microbereich.

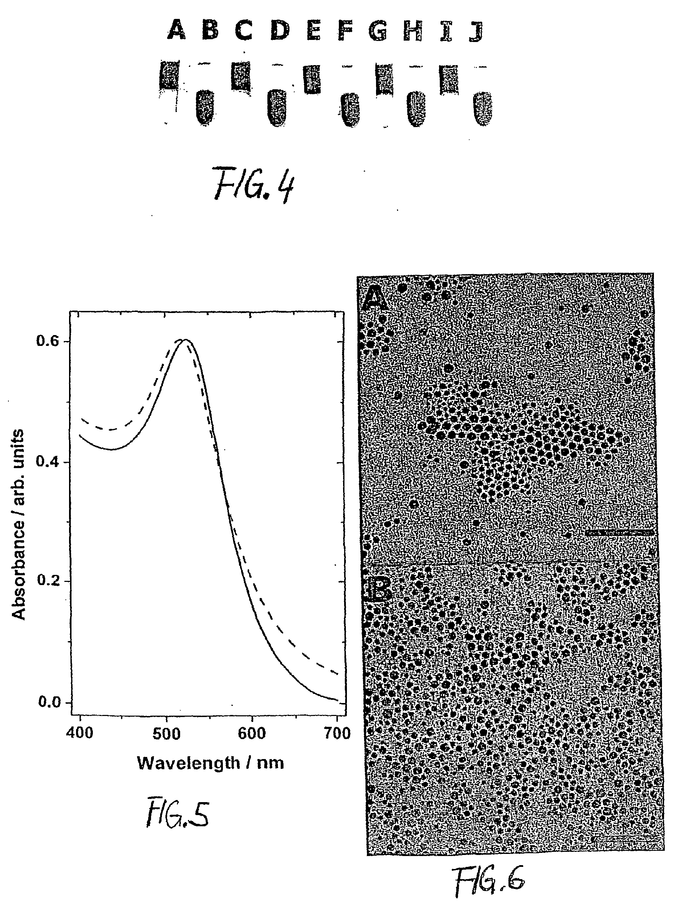

Es wurde ein vollständiger Phasentransfer der metallischen Nanopartikel erreicht, siehe Fig. 2. Dies zeigen auch weitere Ausführungsbeispiele, die ebenfalls einen erfindungsgemäßen Phasentransfer vollzogen haben. Solche Ergebnisse sind in Fig. 4 ff gezeigt. Dabei wurde folgendes experimentelles Setup gewählt:

Zur Nanopartikel-Synthese: eine 30 Millimeter wässrige Lösung eines Metallchlorids (HAuCl₄ oder Na₂PdCl₄, 30 Milliliter) wurde zu einer 25 mM Lösung von Tetraoctylammoniumbromid in Toluol gegeben (80 mL). Der Übergang des Metallsalzes in die Toluolphase konnte innerhalb weniger Sekunden mit dem Auge eindeutig erkannt werden. Eine 0,4 M Lösung von frisch hergestelltem NaBH₄ (25 mL) wurde zur gerührten Mischung gegeben, was das sofortige Einsetzen der Reduktion bewirkte. Nach 30 Minuten wurden die beiden Phasen getrennt, die Toluolphase it 0,1 M H2SO4, 0,1 M NaOH und dreimal mit H2O gewaschen und schließlich über wasserfreiem NaSO4 getrocknet. Frühere, hochaufgelöste TEM-Analysen zeigten, daß auf ähnliche Weise hergestellte Nanopartikel kristallin sind und eine gekappt-oktaedrische Morphologie aufweisen.

Zum Phasentransfer: 1 mL eier wässrigen 0,1 M Lösung von DMAP wurde zu Aliquoten (1 mL) der Nanopartikel-Mischungn gegeben. Diese DMAP-Konzentration erwies sich als ausreichend, um den vollständigen und spontanen Phasentransfer der Nanopartikel zu ermöglichen. Es sollte angemerkt werden, daß es auch gelang, Nanopartikel aus größeren Volumina der Reaktionslösung (bis zu 0,5 L) in Wasser (1mL) zu transferieren und das Tetraalkylammoniumsalz zurückzugewinnen. Der direkte Phasentransfer über die organisch/wässrige Grenze hinweg war innerhalb von einer Stunde vollständig abgelaufen, wobei Rühren oder Schütteln nicht erforderlich war.

Des weiteren ist es möglich, das DMAP direkt zur Toluollösung zu geben, um so die Partikel auszufällen, die dann in Wasser resuspendiert werden konnten. Der Phasentransfer gelang auch mit ähnlichen, in Chloroform synthetisierten Partikeln, nicht aber mit Partikeln, bei deren Synthese andere organische Stabilisierungsmittel verwendet wurden, z. B. Natrium-5,14-diethyl-8,11-dioxo-7,12-dioxaoctadecan-2-sulfonat (Na-AOT) oder Didodecyldimethylammoniumbromid.

Alle Reagenzien wurden von Sigma-Aldrich bezogen und ohne weitere Vorbehandlung eingesetzt. Die UV/Vis-Spektren wurden mit einem UV/Vis-Spektrophotometer von Cary (Modell 4E) aufgenommen, Auflösung 0,2 Nanometer. Die Zeta-Potentiale der Nanopartikel wurden mit einem Zetasizer 4 von Malvern bestimt, wobei das Mittel aus fünf stationären Messungen gebildet wurde. Die Beweglichkeiten wurden mit Hilfe der Smoluchowski-Beziehung in elektrophoretische Potentiale umgerechnet. Die TEM-Untersuchungen wurden mi einem CM12-Mikroskop von Philips bei 120 kV durchgeführt. Die Teilchengrößenverteilung wurde anhand der durch analytisches Ultrazentrifugieren bei 20°C bestimmten Sedimentationsgeschwindigkeiten errechnet. Die Messungen erfolgten mit einer Optima-XL-1-Ultrazentrifuge von Beckman-Coulter, die für die Detektion mit einer Absorptionsoptik ausgerüstet war. Es wurden selbst hergestellte Doppelsektoren-Mittelstücke aus Titan mit einem Durchmesser von 12 Millimetern verwendet. Beim analytischen Ultrazentrifugieren wird auf eine verdünnte Probe der Nanopartikel eine konstante Zentrifugalkraft ausgeübt. Zu Beginn eines Experiments liefert ein Scan bei einer festen Wellenlänge über den Radius der Zelle hinweg einen konstanten Absorptionswert, was eine konstante räumliche Verteilung der Kolloide anzeigt. Im Verlauf des Experiments kann die zeitabhängige Sedimentation der Partikel durch zeitlich aufeinander folgende, radiale Scans der lokalen Kolloidkonzentration verfolgt werden. Die Fraktionierung der Partikel während des Experiments erlaubt es, aus einer zeitlichen Serie radialer Scans die Verteilung der Sedimentationskoeffizienten zu berechnen. Auf diesem Weg lassen sich neben der Dichte des Lösungsmittels und der Viskosität der Lösung auch die Größenverteilung und die Dichte von Partikeln bestimmen, selbst wenn deren Größe im Angströmbereich liegt.

Fig. 4 zeigt nun fünf verdünnte Nanopartikel-Lösungen paarweise von links nach rechts, jeweils vor und nach dem Phasenübergang. Die Partikel sind erkennbar, an der dunkel dargestellten Färbung des Probenbehälter. Hier sind alle Grundfarbendarstellbar, die in Schwarz/weiss Darstellung nicht erkennbar sind.

Es wurden jeweils Phasentransferverfahren durchgeführt aus Reaktionsmischungen (Toluen) unter Verwendung von MUA, wie oben erwähnt in Wasser hinein. Die Beispiele A, B zeigen den Tansfer von Silber-Nanopartikeln, C, D zeigen den Transfer von Gold-Nanopartikeln, E, F den Transfer von Platin-Nanopartikeln, G, H zeigen den Transfer von weiteren Gold-Nanopartikeln, die auf andere Weise hergestellt wurden, und I, J zeigen den Transfer von Palladium-Nanopartikeln.

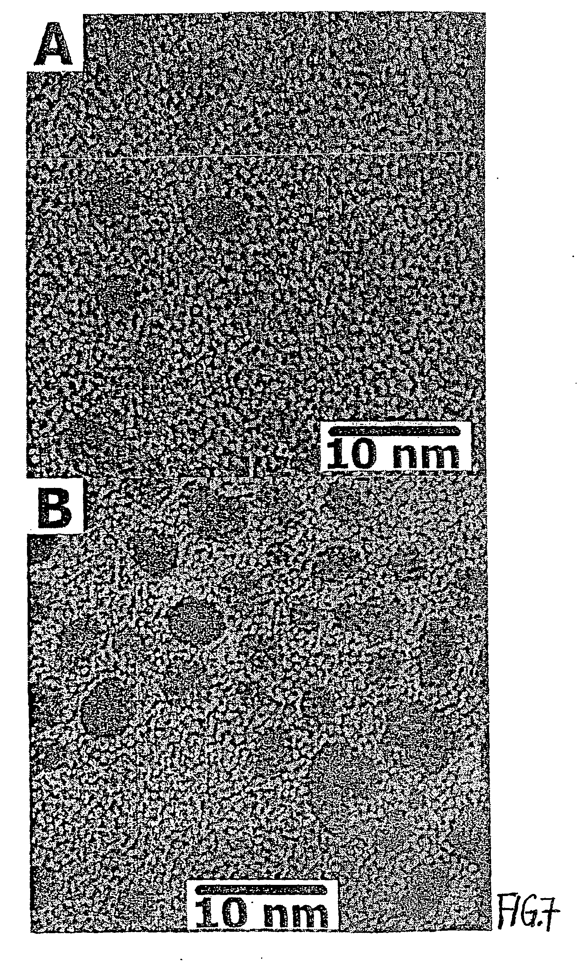

Von den gelösten Gold-Nanopartikeln wurden vor und nach dem Phasentransfer UV/Vis-Spektren aufgenommen, da Partikelaggregationen, reversibel oder irreversibel, Flockulation oder Koagulation, sowie Änderungen in der Dielektrizität der Umgebung der Nanopartikel sich in den optischen Spektren in bekannter Weise bemerkbar machen. In Toluol lag das Maximum der Oberflächenplasmonenbande bei 518 Nanometern Wellenlänge, wie es in Fig. 5 dargestellt ist. Nach dem Phasentransfer war es 6 Nanometer blauverschoben, bei 512 Nanometern. Diese Verschiebung könnte auf der kombinierten Wirkung der Änderung des Brechungsindex des Mediums von 1,47 auf 1,33 und des Austausches der adsorbierten Moleküle beim Transfer beruhen. Jegliche Form der Partikelaggregation würde zu einer Rotverschiebung und Verbreiterung der Plasmonenbandabsorption führen. Die erhaltenen UV/Vis-Spektren zeigen somit eindeutig, daß der DMAP-induzierte Phasentransfer gut dispergierte Gold-Nanopartikel in einer wässrigen Lösung liefert. An den Palladium-Nanopartikeln wurden keine UV-Vis-Experimente durchgeführt, da sie keine starke Oberflächenplasmonenbandabsorption haben.

Fig. 6 zeigt Ergebnisse der Transmissionselektronenmikroskopie (TEM). Die TEM erbrachte keine sichtbaren Unteschiede in den Morphologien der Gold- und Palladium-Nanopartikel nach dem Phasentransfer. Dies geht aus der Zeichnung hervor, wobei A im oberen Bereich in Toluen-synthetisierte Gold-Nanopartikel zeigt, und das Bild B die Probe zeigt, einen Monat nach erfolgtem Transfer in Wasser hinein, der durch die Zugabe von DMAP bewirkt wurde.

Die Analyse der Aufnahmen der Gold-Nanopartikelproben ergab in der Toluol- und in der wässrigen_Lösung einen mittleren Nanopartikeldurchmesser von 5,5 Nanometern mit einer Standardverteilung (SD) von 0,7. Dabei wurden 153 Partikel gezählt, beziehungsweise SD = 0,8 als 115 Partikel gezählt wurden.

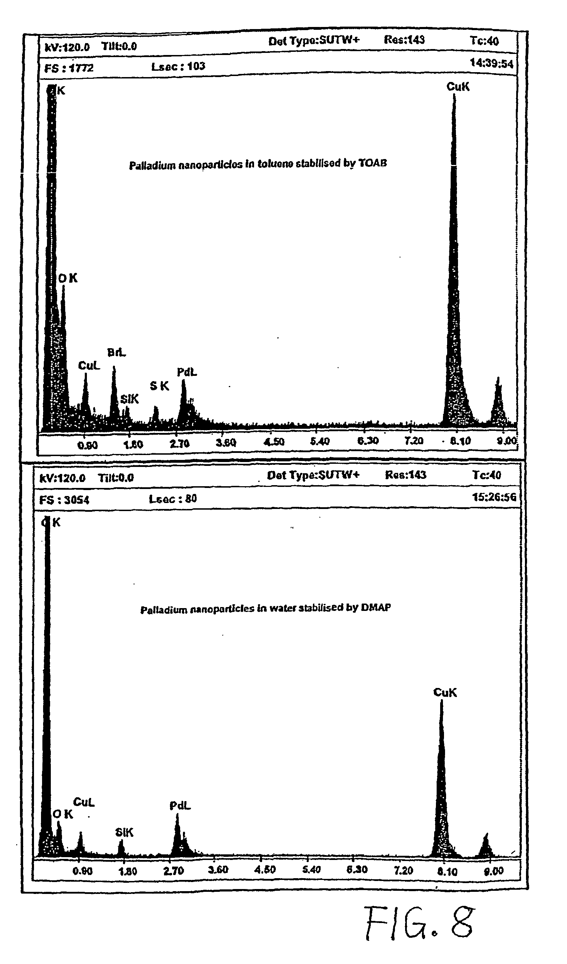

Bei den Palladium-Nanopartikeln ergaben sich mittlere Durchmesser von 4,5 Nanometern, SD = 0,9 bei 145 gezählten Partikeln, beziehungsweise Durchmesser 4,8 Nanometer, SD = 1,2 bei 122 Partikeln. Dies ist in Fig. 7 gezeigt, wobei im oberen Bereich A in Toluen synthetisierte Palladium-Nanopartikel gezeigt sind, und im Bereich B die gleiche Probe nach ihrem Transfer in Wasser gezeigt ist, wobei der Transfer durch die Zugabe von DMAP bewirkt wurde.

Nach der Analyse der energieaufgelösten Röntgenfluoreszenz (EDAX) sind an den in die wässrige Phase übergegangenen, und auf einem TEM-Netz getrockneten Partikeln keine Bromidionen vorhanden. Es sollte hinzugefügt werden, daß die Bromidionen die Gegenionen der Tetraalkylammoniumionen sind. Dennoch können aber noch Spuren des organischen Salzes an der Partikeloberfläche adsorbiert sein.

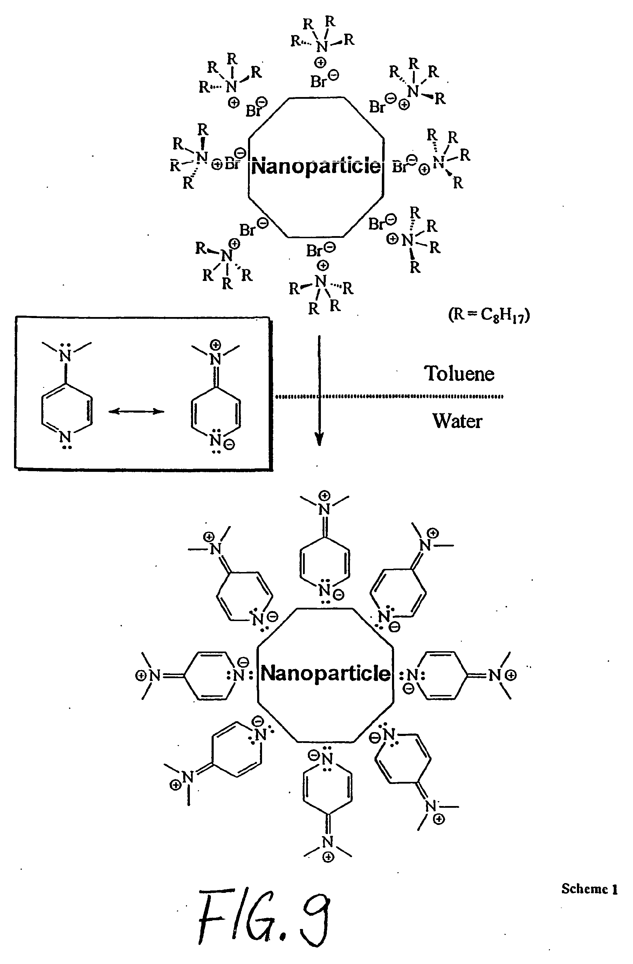

Die EDAX-Spektren von Palladium-Nanopartikeln sind in Fig. 8 gezeigt, oben "wie sythetisiert", und unten nach erfolgtem Phasentransfer in Wasser.

Da sich die aus der TEM erhältlichen Informationen über die Morphologie der Nanopartikel auf den.getrockneten Zustand beziehen, wurden auch Messungen mittels analytischer Ultrazentrifugation (AU) durchgeführt, um die Größenverteilungen der Nanopartikel in Lösung zu erhalten.

Die AU-Analyse der Gold-Nanopartikelproben lieferte mittlere Durchmesser von 5.1 (SD = 1.1) in Toluol und 5,2 nm (SD = 1.1) in Wasser Für die Palladium-Nanopartikel ergab die AU-Analyse Durchmesser von 2.8 (SD = 1,5) bzw. 3.1 nm (SD = 1,6). Diese Ergebnisse stimmen gut mit den durch TEM ermittelten Werten überein und bestätigen nochmals, dass keine wesentliche Aggregation der Nanopartikel infolge des Transfers eingetreten ist.

Die Stabilität der DMAP-stabilisierten Partikel wurde im Hinblick auf Salzkonzentration und pH-Wert untersucht. Alle Proben (Au. Pd. pH 10.5) waren zumindest über einen Zeitraum von 6 Monaten in 3M NaCl-Lösung stabil für Gold = 514 nm). Die Bestimmung des Zeta-Potentials der DMAP-stabilisierten Nanopartikel in wässriger Lösung (pH 10.5) mittls Mikroelektrophorese ergab einen Durchschnittswert von +25 mV (aus fünf Messungen an Proben aus drei verschiedenen Transfer-Experimenten). Dies belegt eine positiv geladene Partikeloberfläche. Die Partikel sind im pH-Bereich von 7 bis 12 kolloidal stabil (mit einem Zeta-Potential von etwa 35 mV), auch wenn der Anteil der flockulierten Partikelzunahme (wie mit dem Auge zu erkenne war; siehe Hintergrundinformationen), wenn der pH-Wert durch die stufenweise Zugabe von verdünnter Säure (1 mM HCl, pH 3) von 10.5 auf 3.0 erniedrigt wurde. Diese Beobachtung ist mit dem postulierten Mechanismus des Phasentransfers (siehe Schema 1) in Einklang, denn die Absenkung des pH-Wertes sollte dazu führen, dass ein größerer Teil der endocyclischen Stickstoffatome protoniert wäre und damit nicht mehr für die Bindung an die Oberfläche der Nanopartikel zur Verfügung stünde, um diese zu stabilisieren. In der Folge würden Bereiche der Nanopartikelober-fläche "entschützt", was zu einer reversiblen Aggregation führen würde. Eine Abnahme des Ausmaßes der Partikel-Flockulation (die an der Blauverschiebung des Peaks der Plasmonenbandabsorption zu erkennen war) wurde durch die Zugabe einer verdünnten Base (1 mM NaOH) erreicht, wodurch der pH-Wert wieder auf seinen ursprünglichen Wert (pH 10.5) erhöht wurde. Die Trennung der aggregierten Partikel erfolgte nicht sofort, sondern konnte erst nach einigen Tagen nachgewiesen werden; der Vorgang war jedoch stets wiederholbar. Wie man auf der Basis einfacher Säure-Base-Gleichgewichte erwarten konnte, führte das Anheben des pH-Wertes der Lösung durch Zugabe einer verdünnten Base auf Werte oberhalb von pH 13 zu einer Aggregation der Partikel, da der Anteil geladener DMAP-Moleküle abnimmt (ph...=9.6).

Um den Mechanismus des spontanen Phasentransfers der metallischen Nanopartikel von der organischen in die wässrige Phase besser zu verstehen, wurde die Wirksamkeit verschiedener Verbindungen erprobt. Diese wurden jeweils als 0,1M wässrige Lösungen zu Aliquoten der Lösung der Gold-Nanooartikel gegeben. Pyridin und 4-Aminopyridin führten zur sofortigen Aggregation der in Toluol suspendierten Partikel, die sich durch einen Farbwechsel von Rot nach Blau und eine nachfolgende Niederschlagsbildung bemerkbar machte. Nur in dem Gefäß, das DMAP enthilet, fand ein Transfer der Nanopartikel in die wässrige Phase statt; bei den anderen Proben sammelte sich der gebildete Niederschlag an der Grenzfläche zwischen Toluol und Wasser. Diese Ergebnisse deuten darauf hin, daß für die Auslösung des Phasentransfers eine tertiäre (stark basische) Aminogruppe in Konjugation zu einer elektronenschiebenden (schwach basischen) Gruppe notwendig ist. Um zu widerlegen, daß sich starke kovalente Bindungen zwischen den stabilisierenden Molekülen (DMAP) und den Oberflächen der metallischen Nanopartikel gebildet haben, wurde die wässrige Phase mehrfach mit Toluol gewaschen. Mit zunehmender Zahl der Extraktionen nahm der Gehalt der wässrigen Phase an DMAP kontinuierlich ab, bis die Partikel schließlich Aggregate bildeten. Dieser Effekt deutet darauf hin, daß das DMAP von den Partikeloberflächen abgelöst wird. Kovalent gebundene Moleküle können nicht durch einfaches Waschen mit einem Lösungsmittel entfernt werden.

Ein möglicher Mechanismus des spontanen Phasentransfers der Nanopartikel in Gegenwart von DMAP-Molekülen ist in Schema 1 in Fig. 9 dargestellt: Die Zugabe einer wässrigen DMAP-Lösung zur Dispersion der Nanopartikel in Toluol führt zu einer Verteilung des DMAP im Wasser-Toluol-Gemisch (dies wurde durch eine dünnschichtchromatographische Analyse der organischen Phase nachgewiesen) und zu dessen Physisorption auf der Oberfläche der Nanopartikel. Einfache Berechnungen zu Säure-Base-Gleichgewichten zeigen, daß in einer 0,1 M wässrigen Lösung 98% der DMAP-Moleküle als freie Base vorliegen. Wir nehmen an, daß die DMAP-Moleküle über die endocyclischen Stickstoffatome mit den Atomen der Metalloberfläche labile Donoar-Acceptor-Komplexe bilden, wie dies schon früher für planare Goldsubstrate beschrieben wurde; für den Übergang in die wässrige Phase ist dann eine Aufladung der Oberfläche erforderlich, die dadurch erreicht werden kann, daß die exocyclischen Stickstoffatome, die von der Oberfläche der Nanopartikel weg zeigen, partiell protoniert werden.

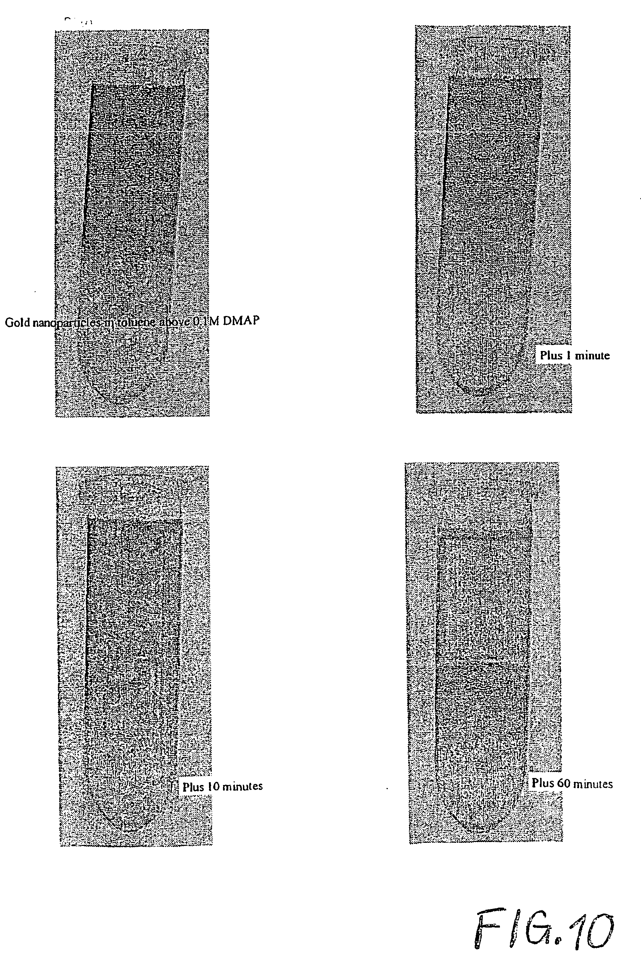

Fig. 10 zeigt Photographien, die den Zeitverlauf beim Phasentransfer zeigen, Goldnanopartikel aus Toluen in 0.1M DMAP Lösung; Links oben sofort bei Einleiten eds Phasentransfers, rechts daneben eine Minute später, wobei die einsetzende Wanderung der Nanopartikel gut erkennbar ist. Links unten nach 10 Minuten, bereits weiter fortgeschritten, und rechts unten nach einer Stunde, praktisch mit abgeschlossenem Phasentransfer.

Es wurde hier eine allgemeine Methode beschrieben, um Gold- und Palladium-Nanopartikel mit hoher Effizienz von einem organischen Lösungsmittel (in diesem Fall Toluol) in Wasser zu überführen.

Dieses Verfahren bietet mindestens drei Vorteile:

Der erste liegt darin, daß es die Hydrosolsynthesemethoden ersetzt, die hohe Verdünnungen und langwierige Dialysereinigungsverfahren erfordern.

Zum zweiten liefern die Synthesen in organischen Lösungsmitteln hohe Konzentrationen an Nanopartikeln mit einer Monodispersität, die deutlich besser ist als die von in Wasser gebildeten Partikeln; die hier beschriebene Methode eröffnet Forschern, deren Experimente auf wässrigen Lösungen basieren, den Zugang zu solchen Partikeln.

Drittens schließlich lassen sich, da der Transfer der Nanopartikel aus der organischen Phase ohne Niederschlagsbildung abläuft, die teuren Ammoniumsalze zurückgewinnen.

Des weiteren können die wasserdispergierbaren metallischen Nanopartikel als Festkörper isoliert werden, was dann wichtig ist, wenn hochkonzentrierte Lösungen der Partikel gebraucht werden, z. B. bei Anwendungen im Bereich kolloidaler Kristalle.

Die zu erwartende starke Affinität der DMAP-stabiliserten Partikel zu negativ geladenen Substraten, wie sie üblicherweise in der heterogenen Katalyse genutzt werden, verdient ebenfalls weitere Untersuchungen.

Gemäß einem weiteren Aspekt der vorliegenden Erfindung kann die wässrige oder alkoholische Lösung, wie sie durch das oben beschriebene Phasentransferverfahren gewonnen wurde, in vorteilhafter Weise auch ganz allgemein dafür verwendet werden, als homogener Katalysatorstoff in Flüssigkeit vorliegende Nanopartikel nach der Grundreaktion wieder aus der Reaktionsflüssigkeit herauszubekommen. Dabei werden beispielsweise Goldnanopartikel als Katalysator einem Reaktionsgemisch zugeführt. Danach erfolgt die chemische Grundreaktion, die durch die Goldnanopartikel katalysiert werden soll. Danach kann der Katalysatorstoff, also hier die Goldnanopartikel, aus dem Reaktionsgemisch erfindungsgemäß wieder entfernt werden:

Dies geschieht durch Zugabe von einem der Stoffe, wie sie aus der oben genannten, generischen Formel ableitbar sind, also beispielsweise durch 4-Dimethyl-Amino-Pyridin vorzugsweise in Lösung in Wasser.

Dieser Stoff bewirkt, wie oben beschrieben, das Wandern des Katalysatorstoffs in das Wasser hinein. Danach kann das nunmehr die Nanopartikel enthaltende Wasser aus dem Reaktionsraum abgetrennt werden. Hieraus ergibt sich der Vorteil, daß eine katalytische Effizienz des Katalysators erreicht wird, die um ein Vielfaches höher liegt als wenn der Katalysatorstoff etwa an Zeolite oder Kohlemoleküle gebunden ist, wie es im Stand der Technik der Fall ist. Dieser Effizienzsprung wird dadurch bewirkt, daß die für die Katalyse wirksame Oberfläche um ein Vielfaches gesteigert wird, bei konstanter Anzahl der verwendeten Katalysatormoleküle, weil die katalysierenden Teilchen homogen in dem Reaktionsgemisch verteilt sind und praktisch mit ihrer gesamten Oberfläche katalytisch wirken können. Es wird daher weit weniger Katalysatorstoff benötigt bei gleicher Wirkung, was die Kosten drastisch senkt, da vor allem Edelmetallnanopartikel sehr teuer sind. Es sei noch angemerkt, dass bei Katalyseverfahren aus dem Stand der Technik, bei denen etwa Zeolite als Katalysatorträgerstoff verwendet wird, ein hoher Anteil von potentiell wirksamer Katalysatorfläche dadurch verloren geht, daß der Katalysatorstoff zu einem wesentlichen Teil mangels Wirkoberfläche inaktiv im Innern des Trägermoleküls bleibt, ohne katalytisch wirken zu können.

Gemäß einem zweiten Vorteil dieses hier vorgestellten homogenen Katalyseverfahrens kann jedoch auch der Katalysatorstoff nach erfolgter Reaktion nahezu zu 100 Prozent wiedergewonnen werden, indem das oben genannte erfinderische Prinzip ausgenutzt wird. Dieses Recycling des Katalysatorstoffs ist zudem auf einfache Weise durchführbar. Auch die trägt zu erheblichen Kostensenkungen bei.

Eine erfindungsgemäße Verfahrensvariante ergibt ein Transferverfahren für Nanopartikel aus alkoholischer oder wäßriger Lösung in organische Lösung. In einem solchen Falle können beispielsweise zum Erhalt reiner Nanopartikel in der organischen Lösung die Nanopartikel, wie oben beschrieben, selektiv abgetrennt werden, indem sie in Wasser überführt werden, wie gemäß dem ersten Hauptaspekt der Erfindung vorgeschlagen wurde. Dann liegt das organische, mit den Nebenprodukten 'verschmutzte' organische Lösungsmittel isoliert vor und kann der Entsorgung bzw. einer anderweitigen Verwendung zugeführt werden. Nun schließen sich die beiden unten erwähnten Schritte an, nämlich Entfernen des Wassers und Zugabe von neuem, nicht-verunreinigtem, organischem Lösungsmittel zu den Nanopartikeln.

Diese Variante enthält im Wesentlichen diese beiden Schritte:
a.) Entfernen eines Anteils des alkoholischen oder wässrigen Lösungsmittels zur Gewinnung der Nanopartikel mit einem reduzierten Gehalt dieses Lösungsmittels,
b.) Zugeben von organischem Lösungsmittel zu den Nanopartikeln.

Diese essentiellen Verfahrensschritte können beispielsweise zur gereinigten Wiederverwendung der Nanopartikel in neuer, frischer organischer Lösung verwendet werden, nachdem solche Nanopartikel in organischer Lösung hergestellt wurden und dabei neben den Nanopartikeln auch unerwünschte Nebenprodukte entstanden sind.

In weiterer Ausbildung der hier offenbarten erfindungsgemässen Lehre können die folgenden 3 verschiedenen Arten von Katalyse, die im Stand der Technik bekannt sind, verbessert werden, indem der erfindungsgemäße Schritt des Phasentransfers als Teil des katalytischen Prozesses verwendet wird. Die 3 Arten sind die folgenden:
1. homogene Katalyse,
2. heterogene Katalyse, und
3. Mischphasen-Katalyse.

### Zur homogenen Katalyse:

Homogene Katalyse beschreibt eine katalytische Reaktion in der der Katalysator, in diesem Falle die Kolloide beziehungsweise Nanopartikel in der Reaktionslösung dispergiert sind. Dies hat den Vorteil, dass die gesamte Nanopartikel-Oberfläche für das Ausgangsmaterial der Reaktion in der Lösung zur Verfügung steht. Die Reaktion kann dann auf oder in der Nähe der Partikeloberfläche stattfinden. Die Reaktionsprodukte sind deshalb in der selben Lösung enthalten wie die Nanopartikel. Dies ist ein Nachteil der homogenen Katalyse nach dem Stand der Technik, weil Nanopartikel nicht auf einfache Weise unter Verwendung von bekannten Trennungstechniken, wie etwa Filtration, aus der Lösung entfernt werden können. Hier setzt die vorliegende Erfindung in zwei Alternativen an:
a) die in einem organischen Lösungsmittel vorliegenden Nanopartikel können als homogene Katalysatoren in den organischen Lösungsmitteln verwendet werden. Nachdem die Reaktion stattgefunden hat, können dann die Nanopartikel aus der Lösung unter Verwendung des erfindungsgemäßen Phasentransfers entfernt werden.
b) in organischem Lösungsmittel vorliegende Nanopartikel können zunächst unter Verwendung des erfindungsgemäßen Phasentransferverfahrens in Wasser mit oder ohne einem gewissen Alkoholgehalt transferiert werden. Danach können die Nanopartikel zur homogenen Katalyse in einem solchen wasserlöslichen Lösungsmittel verwendet werden. Die in die wässrige Lösung transferierten Partikel haben auch verbesserte Eigenschaften gegenüber den in Wasser hergestellten Partikeln, beispielsweise benötigen sie in der Lösung keinen Ko-Katalysator.

### Zur heterogenen Katalyse:

Wie oben beschrieben liegt der Hauptnachteil der homogenen Katalyse in der Tatsache, dass die Trennung des Katalysators (um das Produkt zu recyceln oder zu reinigen) aus der Reaktionsmischung schwierig ist. Wenn jedoch der Katalysator auf einem größeren Objekt fest sitzt, mit einer Dimension größer als 100 Nanometern, dann ist die Trennung wesentlich einfacher. Im Stand der Technik bekannte Träger enthalten Korngrößen im Bereich von Mikrometern. Die vorliegende Erfindung kann die heterogene Katalyse auf folgende Weise verbessern:

In organischen Lösungsmitteln synthetisierte Nanopartikel können in Wasser transferiert werden unter Verwendung des erfindungsgemäßen Phasentransferverfahrens. Die Zugabe einer Trägerverbindung, die so gewählt ist, dass sie eine Affinität für die phasentransferierten Nanopartikel besitzt, zu den in der Lösung befindlichen Nanopartikeln bewirkt, dass die Partikel an die Trägerverbindung anbinden. Die Trägerverbindung kann dann gewaschen und/oder zur Verwendung bei der katalytischen Reaktion auf normale Weise präpariert werden, wie dies im Stand der Technik bekannt ist. Nach erfolgter katalytischer Reaktion können dann die an den Trägern haftenden Nanopartikel unter Verwendung von bekannten Techniken zum Recyceln oder der Trennung aus der Reaktionsmischung wiedergewonnen werden.

### Zur Mischphasen-Katalyse:

Alternativ dazu kann die katalytische Reaktion auch stattfinden, wenn verschiedene Phasen zusammengemischt werden, wobei eine von denen die Nanopartikel als Katalysator enthalten kann. Dabei bedeutet ,Phase' 1 oder mehrere von: Festkörper, Gas oder hydrophiler oder hydrophober Flüssigkeiten. Wenn daher 2 dieser Phasen gemischt werden, so trennen sie sich mit der Zeit. Nanopartikel können dann wie oben beschrieben als Katalysator verwendet werden, wobei das erfindungsgemäße Phasentransferverfahren auf 3 verschiedene Arten verwendet werden kann:
1. bei der Abtrennung des Katalysators nach der Reaktion, beispielsweise in einer Mischung aus hydrophober und hydrophiler Flüssigkeit, oder
2. der Phasentransfer kann dazu verwendet werden, einen Festkörperkatalysator auszubilden der dann beispielsweise in einer Festkörper/Gasmischung verwendet wird, oder
3. um wasserlösliche Nanopartikel zu bilden, die dann am Übergang zu einer anderen Phase reagieren können, beispielsweise am Übergang zwischen hydrophiler Flüssigkeit und Festkörper oder am Übergang zwischen hydrophober und hydrophiler Flüssigkeit in einer entsprechenden Mischung.

Auch Mischformen aller 3 Phasen Festkörper, Flüssigkeit und Gas sind auf entsprechende Weise möglich.

Obwohl die vorliegende Erfindung anhand eines bevorzugten Ausführungsbeispiels vorstehend beschrieben wurde, ist sie darauf nicht beschränkt, sondern auf vielfältige Weise modifizierbar. Insbesondere die hierin gemachten quantitativen Angaben sind nur beispielhaft gedacht und sollen den Schutzumfang nicht beschränken.

Beispielsweise kann beim erfindungsgemässen Phasentransferverfahren der Phasentransfer Katalysator anstatt in wässriger (mit oder ohne Alkoholanteil) befindlich auch isoliert und nicht in Lösung befindlich zugegeben werden. Die Zugabe kann entweder in die Ausgangslösung direkt - etwa als dritte Phase in Pulver- oder Breiform - oder nach Zugabe eines vorbestimmbaren Quantums an Ziellösung erfolgen.

## Patentansprüche

1. Phasentransferverfahren zum Überführen von anorganischen Nanopartikeln aus einer organischen Phase in eine damit nicht mischbare wässrige Phase oder alkoholische Phase unter Verwendung eines Phasentransferkatalysators, der einen Bestandteil Y (12), einen hydrophilen Bestandteil X (16) und einen organischen Spacerbestandteil Z (14) aufweist,
**dadurch gekennzeichnet, dass**
(i) der Bestandteil Y (12) eine schwach basische Gruppe umfasst, der hydrophile Bestandteil X (16) eine stark basische tertiäre Aminogruppe umfasst und diese über den organischen Spacerbestandteil Z (14) in Konjugation zu einander stehen, oder
(ii) der Bestandteil Y (12) eine Thiolgruppe und der hydrophile Bestandteil X (16) eine Carboxygruppe umfasst, oder
(iii) der Phasentransferkatalysator Mercaptopropyltrimethoxysilan ist.

2. Phasentransferverfahren gemäß Anspruch 1, worin der Phasentransferkatalysator gemäß Alternative (i) 4-Dimethylaminopyridin (DMAP) ist.

3. Phasentransferverfahren gemäß Anspruch 1, worin der Phasentransferkatalysator gemäß Alternative (ii) Mercaptoundecansäure (MUA) ist.

4. Verfahren gemäß einem der vorangehenden Ansprüche, worin das Verhältnis der Anzahl der Oberflächenatome eines Nanopartikels zur Anzahl der daran anbindenden Phasentransferkatalysatormoleküle in einem Bereich von 0,1 bis 10 liegt.

5. Verfahren gemäß Anspruch 4, worin dieses Verhältnis 1 beträgt.

6. Phasentransferverfahren gemäß einem der Ansprüche 1 bis 5, worin die Nanopartikel unter Metall-, Metalloxid- und Metalllegierungspartikeln ausgewählt werden.

7. Phasentransferverfahren gemäß Anspruch 6, worin das Metall unter Gold, Silber, Iridium, Platin, Palladium, Nickel, Eisen, Rhodium und Ruthenium ausgewählt wird.

8. Verfahren gemäß Anspruch 6, worin das Metalloxid unter Eisenoxid, Zinkoxid, Titandioxid und Zinnoxid ausgewählt wird.

9. Phasentransferverfahren gemäß einem der Ansprüche 1 bis 5, worin die Nanopartikel Halbleiternanopartikel sind.

10. Phasentransferverfahren gemäß einem der Ansprüche 1 bis 5, worin die Nanopartikel anorganische Nanopartikel sind, die Elemente der seltenen Erden enthalten.

11. Phasentransferverfahren gemäß einem der Ansprüche 1 bis 10, worin die wässrige Phase wasserlösliche Bestandteile, insbesondere Alkohole enthält.

12. Phasentransferverfahren gemäß einem der Ansprüche 1 bis 11, worin nach dem Phasentransfer die wässrige oder alkoholische Phase von der organischen Phase abgetrennt wird.

13. Wässrige oder alkoholische Phase, die eine Lösung von anorganischen Nanopartikeln enthält und einen Phasentransferkatalysator, der an die Oberfläche der Nanopartikel anbinden kann und der einen Bestandteil Y (12), einen hydrophilen Bestandteil X (16) und einen organischen Spacerbestandteil Z (14) aufweist,
**dadurch gekennzeichnet, dass**
(i) der Bestandteil Y (12) eine schwach basische Gruppe umfasst, der hydrophile Bestandteil X (16) eine stark basische tertiäre Aminogruppe umfasst und diese über den organischen Spacerbestandteil Z (14) in Konjugation zu einander stehen.

14. Wässrige oder alkoholische Phase gemäß Anspruch 13, worin der Phasentranferkatalysator 4-Dimethylaminopyridin (DMAP) ist.

15. Wässrige oder alkoholische Phase gemäß Anspruch 13 oder 14, worin die Nanopartikel unter Metall-, Metalloxid- und Metalllegierungspartikeln ausgewählt werden.

16. Wässrige oder alkoholische Phase gemäß Anspruch 15, worin die Metallnanopartikel Goldnanopartikel sind.

17. Verwendung der wässrigen oder alkoholischen Phase gemäß einem der Ansprüche 13 bis 16 in einem Verfahren zum selektiven Beschichten von Oberflächen makroskopischer Körper.

18. Verwendung der wässrigen oder alkoholischen Phase gemäß einem der Ansprüche 13 bis 16 in einem Verfahren zum Beschichten von Trägerpartikeln.

19. Verwendung der wässrigen oder alkoholischen Phase gemäß einem der Ansprüche 13 bis 16 in einem Verfahren zum Markieren von Biomolekülen mit Nanopartikeln.

20. Verwendung der wässrigen oder alkoholischen Phase gemäß einem der Ansprüche 13 bis 16 als (a) Farbe, (b) Druckfarbe, oder (c) Lack.

21. Verwendung der wässrigen oder alkoholischen Phase gemäß einem der Ansprüche 13 bis 16 in einem Verfahren der homogenen, heterogenen Katalyse oder Mischphasenkatalyse.

22. Verwendung gemäß einem der Ansprüche 17 bis 21, worin das Verfahren einen Schritt umfasst, in dem das Lösungsmittel aus der wässrigen oder alkoholischen Phase entfernt wird, unter Erhalt eines Pulvers oder eines Breis.

23. Verwendung gemäß Anspruch 22, worin das Pulver oder der Brei mit einem Lösungsmittel zum Entfernen von Stoffresten gewaschen werden.

## Claims

1. A phase transfer process for transferring inorganic nanoparticles from an organic phase into an aqueous phase or alcoholic phase immiscible therewith using a phase transfer catalyst having a component Y (12), a hydrophilic component X (16) and an organic spacer component Z (14), **characterized in that**:
(i) said component Y (12) comprises a weakly basic group, said hydrophilic component X (16) comprises a strongly basic tertiary amino group, and these two are in conjugation to one another through the organic spacer component Z (14); or
(ii) said component Y (12) comprises a thiol group and said hydrophilic component X (16) comprises a carboxy group; or
(iii) said phase transfer catalyst is mercaptopropyltrimethoxysilane.

2. The phase transfer process according to claim 1, wherein the phase transfer catalyst according to alternative (i) is 4-dimethylaminopyridine (DMAP).

3. The phase transfer process according to claim 1, wherein the phase transfer catalyst according to alternative (ii) is mercaptoundecanoic acid (MUA).

4. The process according to any of the preceding claims, wherein the ratio of the number of surface atoms of a nanoparticle to the number of phase transfer catalyst molecules binding thereto is within a range of from 0.1 to 10.

5. The process according to claim 4, wherein said ratio is 1.

6. The phase transfer process according to any of claims 1 to 5, wherein said nanoparticles are selected from metal, metal oxide and metal alloy particles.

7. The phase transfer process according to claim 6, wherein said metal is selected from gold, silver, iridium, platinum, palladium, nickel, iron, rhodium and ruthenium.

8. The process according to claim 6, wherein said metal oxide is selected from iron oxide, zinc oxide, titanium dioxide and tin oxide.

9. The phase transfer process according to any of claims 1 to 5, wherein said nanoparticles are semiconductor nanoparticles.

10. The phase transfer process according to any of claims 1 to 5, wherein said nanoparticles are inorganic nanoparticles which contain rare earth elements.

11. The phase transfer process according to any of claims 1 to 10, wherein said aqueous phase contains water-soluble components, especially alcohols.

12. The phase transfer process according to any of claims 1 to 11, wherein said aqueous or alcoholic phase is separated from said organic phase after the phase transfer.

13. An aqueous or alcoholic phase which contains a solution of inorganic nanoparticles and a phase transfer catalyst which can bind to the surface of the nanoparticles and includes a component Y (12), a hydrophilic component X (16) and an organic spacer component Z (14), **characterized in that**:
(i) said component Y (12) comprises a weakly basic group, said hydrophilic component X (16) comprises a strongly basic tertiary amino group, and these two are in conjugation to one another through the organic spacer component Z (14).

14. The aqueous or alcoholic phase according to claim 13, wherein said phase transfer catalyst is 4-dimethylaminopyridine (DMAP).

15. The aqueous or alcoholic phase according to claim 13 or 14, wherein said nanoparticles are selected from metal, metal oxide and metal alloy particles.

16. The aqueous or alcoholic phase according to claim 15, wherein said metal nanoparticles are gold nanoparticles.

17. Use of the aqueous or alcoholic phase according to any of claims 13 to 16 in a process for the selective coating of surfaces of macroscopic bodies.

18. Use of the aqueous or alcoholic phase according to any of claims 13 to 16 in a process for the coating of support particles.

19. Use of the aqueous or alcoholic phase according to any of claims 13 to 16 in a process for the labeling of biomolecules with nanoparticles.

20. Use of the aqueous or alcoholic phase according to any of claims 13 to 16 as (a) paint, (b) printing ink or (c) varnish.

21. Use of the aqueous or alcoholic phase according to any of claims 13 to 16 in a process of homogeneous catalysis, heterogeneous catalysis or mixed phase catalysis.

22. The use according to any of claims 17 to 21, wherein said process comprises a step wherein the solvent is removed from the aqueous or alcoholic phase to obtain a powder or slurry.

23. The use according to claim 22, wherein said powder or slurry is washed with a solvent to remove residual substances.

## Revendications

1. Procédé de transfert entre phases, permettant de transporter des nanoparticules inorganiques depuis une phase organique jusque dans une phase aqueuse ou alcoolique non miscible avec celle-là, à l'aide d'un catalyseur par transfert de phase qui comprend un fragment Y (12), un fragment hydrophile X (16) et un fragment écarteur organique Z (14),
**caractérisé en ce que**
a) le fragment Y (12) comporte un groupe faiblement basique, le fragment hydrophile X (14) comporte un groupe amino tertiaire fortement basique, et ces fragments se trouvent conjugués l'un à l'autre par l'intermédiaire du fragment écarteur organique Z (14),
b) ou bien le fragment Y (12) comporte un groupe thiol et le fragment hydrophile X (14) comporte un groupe carboxyle,
c) ou bien le catalyseur par transfert de phase est du mercaptopropyl-triméthoxy-silane.

2. Procédé de transfert entre phases conforme à la revendication 1, dans lequel le catalyseur par transfert de phase, selon l'option (a), est de la 4-diméthylamino-pyridine (DMAP).

3. Procédé de transfert entre phases conforme à la revendication 1, dans lequel le catalyseur par transfert de phase, selon l'option (b), est de l'acide mercapto-undécanoïque (MUA).

4. Procédé conforme à l'une des revendications précédentes, dans lequel le rapport du nombre d'atomes de surface d'une nanoparticule au nombre de molécules de catalyseur par transfert de phase qui s'y lient vaut de 0,1 à 10.

5. Procédé conforme à la revendication 4, dans lequel ledit rapport vaut 1.

6. Procédé de transfert entre phases conforme à l'une des revendications 1 à 5, dans lequel les nanoparticules sont choisies parmi des nanoparticules de métal, d'oxyde de métal et d'alliage de métaux.

7. Procédé de transfert entre phases conforme à la revendication 6, dans lequel le métal est choisi parmi l'or, l'argent, l'iridium, le platine, le palladium, le nickel, le fer, le rhodium et le ruhénium.

8. Procédé de transfert entre phases conforme à la revendication 6, dans lequel l'oxyde de métal est choisi parmi les oxydes de fer, l'oxyde de zinc, le dioxyde de titane et les oxydes d'étain.

9. Procédé de transfert entre phases conforme à l'une des revendications 1 à 5, dans lequel les nanoparticules sont des nanoparticules de semiconducteur.

10. Procédé de transfert entre phases conforme à l'une des revendications 1 à 5, dans lequel les nanoparticules sont des nanoparticules inorganiques contenant des éléments du groupe des terres rares.

11. Procédé de transfert entre phases conforme à l'une des revendications 1 à 10, dans lequel la phase aqueuse contient des constituants hydrosolubles, en particulier des alcools.

12. Procédé de transfert entre phases conforme à l'une des revendications 1 à 11, dans lequel, après le transfert entre phases, on sépare la phase aqueuse ou alcoolique de la phase organique.

13. Phase aqueuse ou alcoolique, qui contient une solution de nanoparticules inorganiques et un catalyseur par transfert de phase qui peut se lier à la surface des nanoparticules et qui comprend un fragment Y (12), un fragment hydrophile X (16) et un fragment écarteur organique Z (14),
**caractérisé en ce que**
a) le fragment Y (12) comporte un groupe faiblement basique, le fragment hydrophile X (14) comporte un groupe amino tertiaire fortement basique, et ces fragments se trouvent conjugués l'un à l'autre par l'intermédiaire du fragment écarteur organique Z (14).

14. Phase aqueuse ou alcoolique, conforme à la revendication 13, dans laquelle le catalyseur par transfert de phase est de la 4-diméthylamino-pyridine (DMAP).

15. Phase aqueuse ou alcoolique, conforme à la revendication 13 ou 14, dans laquelle les nanoparticules sont choisies parmi des nanoparticules de métal, d'oxyde de métal et d'alliage de métaux

16. Phase aqueuse ou alcoolique, conforme à la revendication 15, dans laquelle les nanoparticules sont des nanoparticules d'or.

17. Emploi d'une phase aqueuse ou alcoolique, conforme à l'une des revendications 13 à 16, dans un procédé de revêtement sélectif de surfaces d'objets macroscopiques.

18. Emploi d'une phase aqueuse ou alcoolique, conforme à l'une des revendications 13 à 16, dans un procédé de revêtement de particules de support.

19. Emploi d'une phase aqueuse ou alcoolique, conforme à l'une des revendications 13 à 16, dans un procédé de marquage de biomolécules avec des nanoparticules.

20. Emploi d'une phase aqueuse ou alcoolique, conforme à l'une des revendications 13 à 16, en tant que :
a) colorant,
b) couleur d'impression,
c) ou laque.

21. Emploi d'une phase aqueuse ou alcoolique, conforme à l'une des revendications 13 à 16, dans un procédé de catalyse homogène, de catalyse hétérogène ou de catalyse en phase mixte.

22. Emploi conforme à l'une des revendications 17 à 21, dans lequel le procédé comporte une étape dans laquelle on chasse le solvant de la phase aqueuse ou alcoolique, ce qui permet d'obtenir une poudre ou une pâte.

23. Emploi conforme à la revendication 22, dans lequel on lave la poudre ou la pâte avec un solvant pour en ôter des résidus de matière.
